# EUROPEAN PATENT SPECIFICATION

(11) **EP 2 603 587 B1**
(45) Date of publication and mention of the grant of the patent: **07.10.2015**
(21) Application number: 11740683.5
(22) Date of filing: 08.08.2011
(51) Int. Cl.: C12N 15/113, A61K 31/713, A61P 31/18, A61K 38/46, A61K 31/7088, A61K 31/7105

(54) **PHARMACEUTICAL COMPOSITIONS FOR THE TREATMENT AND PREVENTION OF HIV-1 INFECTIONS**
PHARMAZEUTISCHE ZUSAMMENSETZUNGEN ZUR BEHANDLUNG UND PRÄVENTION VON HIV-1-INFEKTIONEN
COMPOSITIONS PHARMACEUTIQUES POUR LE TRAITEMENT ET PREVENTION DES INFECTIONS PAR LE VIH-1

(30) Priority: 09.08.2010 EP 10305874
(43) Date of publication of application: 19.06.2013
(73) Proprietor: INSERM (Institut National de la Santé et de la Recherche Médicale), 75013 Paris (FR); Institut Pasteur, 75015 Paris (FR); Istituto Nazionale per le Malattie Infettive "La Spallanzani" I.R.C.C.S, 00149 Roma (IT)
(72) Inventor: PERFETTINI, Jean-luc, F-94805 VILLEJUIF (FR); KROEMER, Guido, F-94805 VILLEJUIF (FR); GOUGEON, Marie-Lise, F-75724 PARIS Cedex 15 (FR); PIACENTINI, Mauro, I-00149 ROME (IT)
(74) Representative: Cabinet Plasseraud
(86) International application number: PCT/EP2011/063606
(87) International publication number: WO 2012/019991

(56) References cited:
- EP-A2- 0 183 352
- WO-A1-2005/092099
- WO-A2-03/002528
- WO-A2-03/082193
- US-A- 4 891 221
- US-A- 5 719 132
- US-A1- 2004 110 774
- JORAJURIA SYLVIE ET AL: "ATP binding cassette multidrug transporters limit the anti-HIV activity of zidovudine and indinavir in infected human macrophages.", ANTIVIRAL THERAPY, vol. 9, no. 4, 1 August 2004 (2004-08-01), pages 519-528, XP55016192, ISSN: 1359-6535
- REGIS E G ET AL: "Elevated levels of macrophage migration inhibitory factor (MIF) in the plasma of HIV-1-infected patients and in HIV-1-infected cell cultures: A relevant role on viral replication", VIROLOGY, ACADEMIC PRESS,ORLANDO, US, vol. 399, no. 1, 30 March 2010 (2010-03-30), pages 31-38, XP026923951, ISSN: 0042-6822, DOI: 10.1016/J.VIROL.2009.12.018 [retrieved on 2010-02-24]
- PHILPOTT SEAN ET AL: "CC chemokine receptor 5 genotype and susceptibility to transmission of human immunodeficiency virus type 1 in women.", JOURNAL OF INFECTIOUS DISEASES, vol. 187, no. 4, 15 February 2003 (2003-02-15), pages 569-575, XP002659688, ISSN: 0022-1899
- EUGENIN E A: "Role of Gap Junction and Maxi Channels in Astrocytes During the Pathogenesis of NeuroAIDS.", JOURNAL OF NEUROIMMUNE PHARMACOLOGY, vol. 5, no. Suppl. 1, March 2010 (2010-03) , page S44, XP002659675, & 16TH ANNUAL MEETING FOR THE SOCIETY-ON-NEUROIMMUNE-PHARMACOLOGY; MANHATTAN BEACH, CA, USA; APRIL 14 -17, 2010
- BARAT CORINNE ET AL: "Extracellular ATP reduces HIV-1 transfer from immature dendritic cells to CD4+ T lymphocytes", RETROVIROLOGY, BIOMED CENTRAL LTD., LONDON, GB, vol. 5, no. 1, 28 March 2008 (2008-03-28), page 30, XP021038031, ISSN: 1742-4690
- LEAL D B R ET AL: "HIV infection is associated with increased NTPDase activity that correlates with CD39-positive lymphocytes", BIOCHIMICA ET BIOPHYSICA ACTA. MOLECULAR CELL RESEARCH, ELSEVIER SCIENCE PUBLISHERS, AMSTERDAM, NL, vol. 1746, no. 2, 15 December 2005 (2005-12-15), pages 129-134, XP025256164, ISSN: 0167-4889, DOI: 10.1016/J.BBAMCR.2005.10.009 [retrieved on 2005-12-15]
- BARAT ET AL: "The Nucleoside Triphosphate Diphosphohydrolase-1/CD39 Is Incorporated into Human Immunodeficiency Type 1 Particles, Where It Remains Biologically Active", JOURNAL OF MOLECULAR BIOLOGY, ACADEMIC PRESS, UNITED KINGDOM, vol. 371, no. 1, 9 July 2007 (2007-07-09), pages 269-282, XP022145907, ISSN: 0022-2836, DOI: 10.1016/J.JMB.2007.05.012
- HARMON BROOKE ET AL: "Induction of the Galpha(q) signaling cascade by the human immunodeficiency virus envelope is required for virus entry", FASEB JOURNAL, FED. OF AMERICAN SOC. FOR EXPERIMENTAL BIOLOGY, US, vol. 82, no. 18, 1 September 2008 (2008-09-01), pages 9191-9205, XP002570739, ISSN: 0892-6638, DOI: 10.1128/JVI.00424-08 [retrieved on 2008-07-16]
- ANAND APPAKKUDAL R ET AL: "HIV-1 gp120-induced migration of dendritic cells is regulated by a novel kinase cascade involving Pyk2, p38 MAP kinase, and LSP1.", BLOOD 22 OCT 2009 LNKD- PUBMED:19700666, vol. 114, no. 17, 22 October 2009 (2009-10-22), pages 3588-3600, XP002659674, ISSN: 1528-0020

## Description

### FIELD OF THE INVENTION:

The present disclosure relates to a blocking agent of a component of the purinergic signalling pathway selected from the group consisting of pannexin 1, ATP or P2Y2 receptor for use in the treatment or prevention of HIV-1 infection.

### BACKGROUND OF THE INVENTION:

Acquired immunodeficiency syndrome (AIDS) refers to an array of symptoms resulting from damage to the immune system by the human immunodeficiency virus type 1 (HIV-1). By reducing the effectiveness of the immune system, AIDS renders infected individuals susceptible to opportunistic infections and otherwise rare tumors. Over 33 million people live with the disease worldwide and there have been 25 million fatal cases so far. Between 3 and 4 million people are newly infected and over 2 million people die per year, including 330,000 children. While highly active anti-retroviral therapy (HAART) for AIDS can slow the progression of disease, there is currently no vaccine or cure for infection. Due to repeated recent failures to develop a vaccine, there is a growing consensus that more basic research will be required to understand pathogenesis caused by HIV infection before effective treatment can be developed.

Infection with the human immunodeficiency virus type 1 (HIV-1) is characterized by viral replication and the gradual loss of CD4⁺ T cells, which are associated with a progressive immunodeficiency that lead to opportunistic infections, neoplasms and ultimately death. The mechanisms that trigger viral dissemination and CD4⁺ T-cell death are still not fully understood, even though the magnitude of apoptosis observed in HIV-infected individuals correlates with the different stages of the disease. Several non exclusive hypotheses have been suggested to explain this T cell depletion in HIV disease. Apoptotic destruction of infected cells ('direct killing') and of uninfected cells which are immunologically relevant ('bystander killing') participate in T cell demise and contribute to viral spread. Viral proteins (Env, Nef, Tat, Vpr) and host factors (overexpression of Fas/Apo-1/CD95 on the surface of CD4⁺ or CD8⁺ cells, overexpression of TRAIL, decreased BCL-2 expression among the CD8⁺ cells, imbalance of Th1/Th2 cytokine profile, oxidative stress) all participate in the immunopathology of HIV-1 infection.

The HIV-1 envelope (Env) glycoprotein complex (gp10/gp41) present in the membrane of viral particles or HIV-1-infected cells triggers the juxtaposition of and later fusion with target cell membranes containing Env receptors and coreceptors (CD4 and CXCR4/CCR5). This process involves the formation of an adhesive junction at the contact site between Env-exposing and CD4 plus CXCR4/CCR5-expressing membranes. This two-membrane structure - also called virological synapse (VS) or infectious synapse (IS) - is similar to the immunological synapse observed during T cell/T cell and during T cell/dendritic cell interactions and requires the production of nanotubes, as well as the recruitment in a supra-molecular complex of Env, co-receptors (CD4, CXCR4, CCR5), integrins (e.g. LFA1, α4β7), tetraspanins (e.g. CD81), adhesion proteins (e.g. ICAM-1, ICAM-3), and intracellular proteins associated with signaling pathways (e.g. TALIN) or cytoskeleton (eg tubulin and actin). Most if not all HIV transmission occurs via this cell-to-cell interaction across this virological synapse. Although the fusogenic activity of Env supports HIV-1 replication, it may also stimulate bystander cell death. Mutation in the fusion domain of gp41 resulted in a fusion-defective Env that failed to induce apoptosis. In addition, an Env protein in which the loop region of gp41 was mutated conserved the capacity to catalyze lipid transfer (hemifusion) to bystander cells but became defective in cell-to-cell fusion. This mutant was still able to induce apoptosis in bystander cells, suggesting that gp41-mediated hemifusion is both required and sufficient for induction of apoptosis in bystander cells. When the fusion between the two interacting cells allow the formation of a fusion pore, multinucleated giant cells are produced - also called syncytia - and finally, syncytia succumbs through apoptosis. This mode of cell death induction likewise participates in the HIV-1-induced immunopathology because syncytia can be found in lymphoid tissue from AIDS patients. Moreover, the presence of syncytia, so-called "multinuclear giant cells", is pathognonomic for the HIV-1 induced encephalitis ("neuro-AIDS").

Therefore, there is an incentive to identify the molecules involved in the formation and maintenance of the virological synapse. By improving knowledge of the molecular mechanisms responsible for such a synapse this may help to identify novel therapeutic targets and develop new approaches that could improve the treatment of HIV-1 infection.

WO 03/082193 discloses the antiviral activity of suramin and its use for the prevention of HIV infection.

US 2004/110774 discloses the use of P2X purinoceptor antagonists for treating HIV infections.

WO 2005/092099 discloses the use of bicyclic uracil derivatives for preventing or treating infection by HIV.

### SUMMARY OF THE INVENTION

The present invention is defined by the claims.

### DETAILED DESCRIPTION OF THE INVENTION

Extracellular adenosine triphosphate (ATP) can activate purinergic receptors of the plasma membrane and modulate multiple cellular functions. Here, the inventors observed that ATP is released from HIV-1 target cells through pannexin-1 channels upon interaction between the HIV-1 envelope protein and specific host cell receptors. Extracellular ATP then acts on purinergic receptors, in particular P2Y2 receptors, to stimulate a signal transduction pathway that involves Pyk2 kinase activation and transient plasma membrane depolarization, which in turn stimulates fusion between Env-expressing membranes and membranes containing CD4 plus appropriate chemokine co-receptors. Inhibition of each of the protein constituents of this cascade (pannexin-1→ATP→P2Y2→Pyk2) can inhibit the replication of HIV-1 mutants that are resistant to conventional antiretroviral agents. Altogether our results delineate a novel signaling pathway involved in early steps of HIV-1 infection that offers ample opportunities for the development of new therapeutic approaches.

### Therapeutic methods and use:

The present disclosure provides methods and compositions (such as pharmaceutical compositions) for treating or preventing human immunodeficiency virus type 1 (HIV-1) infection.

According to a first aspect, the invention relates to a blocking agent of a component of the purinergic signalling pathway which is the P2Y2 receptor for use in the treatment or prevention of HIV-1 infection.

As used herein, the term "purinergic signalling pathway" include the intracellular and extracellular components and events that are involved in the extracellular release of purines (e.g. ATP, ADP, UTP, UDP) and/or the generation, amplification, transmission and subsequent inhibition of a cellular signal induced by purines (e.g. ATP, ADP, UTP, UDP) to the cellular and nuclear machinery responsible for bringing about a change in cell behaviour. The component of the purinergic signalling pathway according to the disclosure includes ATP, the purinergic receptors, pannexin 1 and PYK2.

According to the invention, the blocking agent of a component of the purinergic signalling pathway may be selected from the group consisting of P2Y2 receptor antagonists.

As use herein, the term "purinergic receptor" has its general meaning in the art and refers to P1 and P2 receptors. "P1 receptors" also named as "adenosine receptors" are a class of purinergic receptors, G-protein coupled receptors with adenosine as endogenous ligand ("P2 receptors" comprise P2X and P2Y receptors. "P2Y receptors" are a family of purinergic receptors, G protein-coupled receptors stimulated by nucleotides such as ATP, ADP, UTP, UDP and UDP-glucose. To date, twelve P2Y receptors have been cloned in humans: P2Y1, P2Y2, P2Y4, P2Y5, P2Y6, P2Y8, P2Y9, P2Y10, P2Y11, P2Y12, P2Y13 and P2Y14 (Abbracchio MP, Burnstock G, Boeynaems JM, Barnard EA, Boyer JL, Kennedy C, Knight GE, Fumagalli M, Gachet C, Jacobson KA, Weisman GA (2006). "International Union of Pharmacology LVIII: update on the P2Y G protein-coupled nucleotide receptors: from molecular mechanisms and pathophysiology to therapy". Pharmacol. Rev. 58 (3): 281-341).

The term "P2Y2 receptor antagonist" includes any chemical or biological entity that, upon administration to a subject, results in inhibition or down-regulation of a biological activity associated with activation of the P2Y2 receptor in the subject, including any of the downstream biological effects otherwise resulting from the binding to P2Y2 receptor of its natural ligand (e.g. ATP). Such P2Y2 receptor antagonists include any agent that can block P2Y2 receptor activation or any of the downstream biological effects of P2Y2 receptor activation. For example, such a P2Y2 receptor antagonist can act by occupying the binding site or a portion thereof of the P2Y2 receptor, thereby making the receptor inaccessible to its natural ligand (e.g. ATP) so that its normal biological activity is prevented or reduced. The antagonistic activity of compounds towards the P2Y2 receptors may be determined using various methods well known in the art. For example, the agents may be tested for their capacity to block the interaction of P2Y2 receptor with a natural ligand of P2Y2 receptor (e.g. ATP). For example, the assay is performed with P2Y2 receptor expressed on the surface of cells. A typical assay for determining the antagonistic activities of a compound on P2Y2 receptor is described in P. Hillmann, G.-Y. Ko, A. Spinrath, A. Raulf, I. von Kügelgen, S.C. Wolff, R.A. Nicholas, E. Kostenis, H.-D. Höltje and C.E. Müller, J. Med. Chem. 52 (2009), p. 27620. Briefly, the potency of the test compounds to inhibit UTP-induced intracellular calcium release in NG108-15 cells (mouse neuroblastoma x rat glioma hybrid cell line) may be determined by a fluorescence method using the calcium-chelating fluorophor Oregon Green®.

As used herein, the term "pannexin 1" or "Panx1" has its general meaning in the art and refers to the mechanosensitive ion channel pannexin 1.

The term "pannexin 1 antagonist" includes any chemical or biological entity that, upon administration to a subject, results in inhibition or down-regulation of a biological activity associated with activation of pannexin 1 in the subject, including any of the downstream biological effects. Such pannexin 1 antagonists include any agent that can block the release of ATP by pannexin 1 or any of the downstream biological effects resulting from said release. For example, such a pannexin 1 antagonist can act by occupying the channel or a portion thereof, thereby avoiding the release of ATP so that its normal biological activity is prevented or reduced. The antagonistic activity of compounds towards pannexin 1 may be determined using various methods well known in the art. For example, the agents may be tested for their capacity to block the release of ATP by the channel. Typically, the assay is performed with pannexin 1 expressed on the surface of cells.

A blocking agent of the disclosure may be a low molecular weight antagonist, e. g. a small organic molecule. The term "small organic molecule" refers to a molecule of a size comparable to those organic molecules generally used in pharmaceuticals. The term excludes biological macromolecules (e. g., proteins, nucleic acids, etc.). Preferred small organic molecules range in size up to about 5000 Da, more preferably up to 2000 Da, and most preferably up to about 1000 Da.

Exemplary small organic molecules that are P2Y2 receptor antagonists include but are not limited to uracil nucleotide analogs as described in Sauer R et al. (Sauer R, El-Tayeb A, Kaulich M, Müller CE.Synthesis of uracil nucleotide analogs with a modified, acyclic ribose moiety as P2Y(2) receptor antagonists Bioorg Med Chem. 2009 Jul 15;17(14):5071-9. Epub 2009 May 30) or 4-Phenylamino-substituted 1-amino-2-sulfoanthraquinones as described in Weyler S. et al. (Weyler S, Baqi Y, Hillmann P, Kaulich M, Hunder AM, Müller IA, Müller CE. Combinatorial synthesis of anilinoanthraquinone derivatives and evaluation as non-nucleotide-derived P2Y2 receptor antagonists. Bioorg Med Chem Lett. 2008 Jan 1;18(1):223-7. Epub 2007 Oct 30.). Typical P2Y2 receptor antagonists are diphosphoric 5-(2,4-dioxo-3,4-dihydropyrimidin-1(2H)-yl)pentylphosphonic anhydride, 4-phenyl-amino-4-(2-methoxyphenyl)-2-sulfoanthraquinone (PSB-716).

Exemplary small organic molecules that are pannexin 1 antagonists include but are not limited to 4-(dipropylsulfamoyl)benzoic acid also known as Probenecid, benzoylbenzoyl-ATP, brilliant blue G and carbenoxolone.

In one embodiment, the blocking agent of the invention may consist in an antibody (the term including antibody fragment) directed against the P2Y2 receptor, in such a way that said antibody impairs the activation of said receptor.

Antibodies can be raised according to known methods by administering the appropriate antigen or epitope to a host animal selected, e.g., from pigs, cows, horses, rabbits, goats, sheep, and mice, among others. Various adjuvants known in the art can be used to enhance antibody production. Although antibodies useful in practicing the invention can be polyclonal, monoclonal antibodies are preferred. Monoclonal antibodies can be prepared and isolated using any technique that provides for the production of antibody molecules by continuous cell lines in culture. Techniques for production and isolation include but are not limited to the hybridoma technique; the human B-cell hybridoma technique; and the EBV-hybridoma technique. Alternatively, techniques described for the production of single chain antibodies (see, e.g., U.S. Pat. No. 4,946,778) can be adapted to produce anti-P2Y2 receptor single chain antibodies. The blocking agents of the invention also include anti-P2Y2 receptorantibody fragments including but not limited to F(ab')₂ fragments, which can be generated by pepsin digestion of an intact antibody molecule, and Fab fragments, which can be generated by reducing the disulfide bridges of the F(ab')₂ fragments. Alternatively, Fab and/or scFv expression libraries can be constructed to allow rapid identification of fragments having the desired specificity to the receptor.

Humanized antibodies and antibody fragments therefrom can also be prepared according to known techniques. "Humanized antibodies" are forms of non-human (e.g., rodent) chimeric antibodies that contain minimal sequence derived from non-human immunoglobulin. For the most part, humanized antibodies are human immunoglobulins (recipient antibody) in which residues from a hypervariable region (CDRs) of the recipient are replaced by residues from a hypervariable region of a non-human species (donor antibody) such as mouse, rat, rabbit or nonhuman primate having the desired specificity, affinity and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. Methods for making humanized antibodies are described, for example, by Winter (U.S. Pat. No. 5,225,539) and Boss (Celltech, U.S. Pat. No. 4,816,397).

Then after raising antibodies as above described, the skilled man in the art can easily select those blocking the P2Y2 receptor.

In another embodiment the blocking agent of the invention is an aptamer directed against the P2Y2 receptor. Aptamers are a class of molecule that represents an alternative to antibodies in term of molecular recognition. Aptamers are oligonucleotide or oligopeptide sequences with the capacity to recognize virtually any class of target molecules with high affinity and specificity. Such ligands may be isolated through Systematic Evolution of Ligands by Exponential enrichment (SELEX) of a random sequence library. The random sequence library is obtainable by combinatorial chemical synthesis of DNA. In this library, each member is a linear oligomer, eventually chemically modified, of a unique sequence. Peptide aptamers consists of a conformationally constrained antibody variable region displayed by a platform protein, such as E. coli Thioredoxin A that are selected from combinatorial libraries by two hybrid methods.

Then after raising aptamers directed against the P2Y2 receptor as above described, the skilled man in the art can easily select those blocking the said receptor.

In another embodiment, the blocking agent according to the invention is an inhibitor of P2Y2 expression selected from the group consisting of antisense RNA or DNA molecules, small inhibitory RNAs (siRNAs), short hairpin RNA and ribozymes.

An "inhibitor of gene expression" refers to a natural or synthetic compound that has a biological effect to inhibit or significantly reduce the expression of a gene. Consequently, said inhibitor of gene expression may be an inhibitor of P2Y2 receptor gene expression.

Inhibitors of gene expression for use in the present invention may be based on anti-sense oligonucleotide constructs. Anti-sense oligonucleotides, including anti-sense RNA molecules and anti-sense DNA molecules, would act to directly block the translation of the mRNA by binding thereto and thus preventing protein translation or increasing mRNA degradation, thus decreasing the level of the protein P2Y2 receptor), and thus activity, in a cell. For example, antisense oligonucleotides of at least about 15 bases and complementary to unique regions of the mRNA transcript sequence encoding the targeted protein (e.g. P2Y2 receptor) can be synthesized, e.g., by conventional phosphodiester techniques and administered by e.g., intravenous injection or infusion. Methods for using antisense techniques for specifically inhibiting gene expression of genes whose sequence is known are well known in the art (e.g. see U.S. Pat. Nos. 6,566,135; 6,566,131; 6,365,354; 6,410,323; 6,107,091; 6,046,321; and 5,981,732).

Small inhibitory RNAs (siRNAs) can also function as inhibitors of gene expression for use in the present invention. Gene expression can be reduced by contacting a subject or cell with a small double stranded RNA (dsRNA), or a vector or construct causing the production of a small double stranded RNA, such that gene expression is specifically inhibited (i.e. RNA interference or RNAi). Methods for selecting an appropriate dsRNA or dsRNA-encoding vector are well known in the art for genes whose sequence is known (e.g. see Tuschl, T. et al. (1999); Elbashir, S. M. et al. (2001); Hannon, GJ. (2002); McManus, MT. et al. (2002); Brummelkamp, TR. et al. (2002); U.S. Pat. Nos. 6,573,099 and 6,506,559; and International Patent Publication Nos. WO 01/36646, WO 99/32619, and WO 01/68836).

### Exemplary siRNAs against PYK2 are described in EXAMPLE.

Ribozymes can also function as inhibitors of gene expression for use in the present invention. Ribozymes are enzymatic RNA molecules capable of catalyzing the specific cleavage of RNA. The mechanism of ribozyme action involves sequence specific hybridization of the ribozyme molecule to complementary target RNA, followed by endonucleolytic cleavage. Engineered hairpin or hammerhead motif ribozyme molecules that specifically and efficiently catalyze endonucleolytic cleavage of mRNA sequences are thereby useful within the scope of the present invention. Specific ribozyme cleavage sites within any potential RNA target are initially identified by scanning the target molecule for ribozyme cleavage sites, which typically include the following sequences, GUA, GUU, and GUC. Once identified, short RNA sequences of between about 15 and 20 ribonucleotides corresponding to the region of the target gene containing the cleavage site can be evaluated for predicted structural features, such as secondary structure, that can render the oligonucleotide sequence unsuitable. The suitability of candidate targets can also be evaluated by testing their accessibility to hybridization with complementary oligonucleotides, using, e.g., ribonuclease protection assays.

Both antisense oligonucleotides and ribozymes useful as inhibitors of gene expression can be prepared by known methods. These include techniques for chemical synthesis such as, e.g., by solid phase phosphoramadite chemical synthesis. Alternatively, anti-sense RNA molecules can be generated by in vitro or in vivo transcription of DNA sequences encoding the RNA molecule. Such DNA sequences can be incorporated into a wide variety of vectors that incorporate suitable RNA polymerase promoters such as the T7 or SP6 polymerase promoters. Various modifications to the oligonucleotides of the invention can be introduced as a means of increasing intracellular stability and half-life. Possible modifications include but are not limited to the addition of flanking sequences of ribonucleotides or deoxyribonucleotides to the 5' and/or 3' ends of the molecule, or the use of phosphorothioate or 2'-O-methyl rather than phosphodiesterase linkages within the oligonucleotide backbone.

Antisense oligonucleotides siRNAs and ribozymes of the invention may be delivered in vivo alone or in association with a vector. In its broadest sense, a "vector" is any vehicle capable of facilitating the transfer of the antisense oligonucleotide siRNA or ribozyme nucleic acid to the cells and preferably cells expressing the targeted proteins (P2Y2 receptor). Preferably, the vector transports the nucleic acid to cells with reduced degradation relative to the extent of degradation that would result in the absence of the vector. In general, the vectors useful in the invention include, but are not limited to, plasmids, phagemids, viruses, other vehicles derived from viral or bacterial sources that have been manipulated by the insertion or incorporation of the antisense oligonucleotide siRNA or ribozyme nucleic acid sequences. Viral vectors are a preferred type of vector and include, but are not limited to nucleic acid sequences from the following viruses: retrovirus, such as moloney murine leukemia virus, harvey murine sarcoma virus, murine mammary tumor virus, and rouse sarcoma virus; adenovirus, adeno-associated virus; SV40-type viruses; polyoma viruses; Epstein-Barr viruses; papilloma viruses; herpes virus; vaccinia virus; polio virus; and RNA virus such as a retrovirus. One can readily employ other vectors not named but known to the art.

Preferred viral vectors are based on non-cytopathic eukaryotic viruses in which non-essential genes have been replaced with the gene of interest. Non-cytopathic viruses include retroviruses (e.g., lentivirus), the life cycle of which involves reverse transcription of genomic viral RNA into DNA with subsequent proviral integration into host cellular DNA. Retroviruses have been approved for human gene therapy trials. Most useful are those retroviruses that are replication-deficient (i.e., capable of directing synthesis of the desired proteins, but incapable of manufacturing an infectious particle). Such genetically altered retroviral expression vectors have general utility for the high-efficiency transduction of genes in vivo. Standard protocols for producing replication-deficient retroviruses (including the steps of incorporation of exogenous genetic material into a plasmid, transfection of a packaging cell lined with plasmid, production of recombinant retroviruses by the packaging cell line, collection of viral particles from tissue culture media, and infection of the target cells with viral particles) are provided in Kriegler, 1990 and in Murry, 1991).

Preferred viruses for certain applications are the adeno-viruses and adeno-associated viruses, which are double-stranded DNA viruses that have already been approved for human use in gene therapy. The adeno-associated virus can be engineered to be replication deficient and is capable of infecting a wide range of cell types and species. It further has advantages such as, heat and lipid solvent stability; high transduction frequencies in cells of diverse lineages, including hemopoietic cells; and lack of superinfection inhibition thus allowing multiple series of transductions. Reportedly, the adeno-associated virus can integrate into human cellular DNA in a site-specific manner, thereby minimizing the possibility of insertional mutagenesis and variability of inserted gene expression characteristic of retroviral infection. In addition, wild-type adeno-associated virus infections have been followed in tissue culture for greater than 100 passages in the absence of selective pressure, implying that the adeno-associated virus genomic integration is a relatively stable event. The adeno-associated virus can also function in an extrachromosomal fashion.

Other vectors include plasmid vectors. Plasmid vectors have been extensively described in the art and are well known to those of skill in the art. See e.g. Sambrook et al., 1989. In the last few years, plasmid vectors have been used as DNA vaccines for delivering antigen-encoding genes to cells in vivo. They are particularly advantageous for this because they do not have the same safety concerns as with many of the viral vectors. These plasmids, however, having a promoter compatible with the host cell, can express a peptide from a gene operatively encoded within the plasmid. Some commonly used plasmids include pBR322, pUC18, pUC19, pRC/CMV, SV40, and pBlueScript. Other plasmids are well known to those of ordinary skill in the art. Additionally, plasmids may be custom designed using restriction enzymes and ligation reactions to remove and add specific fragments of DNA. Plasmids may be delivered by a variety of parenteral, mucosal and topical routes. For example, the DNA plasmid can be injected by intramuscular, intradermal, subcutaneous, or other routes. It may also be administered by intranasal sprays or drops, rectal suppository and orally. It may also be administered into the epidermis or a mucosal surface using a gene-gun. The plasmids may be given in an aqueous solution, dried onto gold particles or in association with another DNA delivery system including but not limited to liposomes, dendrimers, cochleate and microencapsulation.

For the purposes of the disclosure, the term "ATP-degrading agent" is intended to mean a compound (natural or not) that has the capability to degrade the extracellular ATP. Typically, an ATP-degrading agent according to the disclosure is an enzyme such as a nucleotide triphosphate degrading enzyme (apyrase). In another embodiment, the ATP-degrading agent is an agent which stimulates the ATPase present at the cell membrane such as CD39. In particular, said agent may consist in a small organic molecule, an antibody or an aptamer that may be obtained as above described.

The properties of the blocking agents of the disclosure make them especially useful in the prophylaxis and treatment of HIV-1 infection.

Accordingly, another object of the disclosure relates to a method for treating or preventing HIV-1 infection comprising administering a subject in need thereof with a blocking agent as above described.

As used herein, the term "subject" denotes a mammal, such as a rodent, a feline, a canine, and a primate. Preferably, a subject according to the invention is a human.

Blocking agents of the invention may be administered in the form of a pharmaceutical composition, as defined below.

Preferably, said antagonist or inhibitor is administered in a therapeutically effective amount.

By a "therapeutically effective amount" is meant a sufficient amount of the blocking agent to treat and/or to prevent HIV-1 infections at a reasonable benefit/risk ratio applicable to any medical treatment.

It will be understood that the total daily usage of the compounds and compositions of the present invention will be decided by the attending physician within the scope of sound medical judgment. The specific therapeutically effective dose level for any particular subject will depend upon a variety of factors including the disorder being treated and the severity of the disorder; activity of the specific compound employed; the specific composition employed, the age, body weight, general health, sex and diet of the subject; the time of administration, route of administration, and rate of excretion of the specific compound employed; the duration of the treatment; drugs used in combination or coincidental with the specific polypeptide employed; and like factors well known in the medical arts. For example, it is well within the skill of the art to start doses of the compound at levels lower than those required to achieve the desired therapeutic effect and to gradually increase the dosage until the desired effect is achieved. However, the daily dosage of the products may be varied over a wide range from 0.01 to 1,000 mg per adult per day. Preferably, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the subject to be treated. A medicament typically contains from about 0.01 mg to about 500 mg of the active ingredient, preferably from 1 mg to about 100 mg of the active ingredient. An effective amount of the drug is ordinarily supplied at a dosage level from 0.0002 mg/kg to about 20 mg/kg of body weight per day, especially from about 0.001 mg/kg to 7 mg/kg of body weight per day.

### Pharmaceutical compositions:

The blocking agent of the invention may be combined with pharmaceutically acceptable excipients, and optionally sustained-release matrices, such as biodegradable polymers, to form therapeutic compositions.

The term "Pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

In the pharmaceutical compositions of the present invention, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms.

Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Solutions comprising compounds of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The blocking agent of the invention can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active polypeptides in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion. Some variation in dosage will necessarily occur depending on the condition of the subject being treated. The person responsible for administration will, in any event, determine the appropriate dose for the individual subject.

The blocking agent of the invention may be formulated within a therapeutic mixture to comprise about 0.0001 to 1.0 milligrams, or about 0.001 to 0.1 milligrams, or about 0.1 to 1.0 or even about 10 milligrams per dose or so. Multiple doses can also be administered.

In addition to the compounds of the invention formulated for parenteral administration, such as intravenous or intramuscular injection, other pharmaceutically acceptable forms include, e.g. tablets or other solids for oral administration; liposomal formulations; time release capsules ; and any other form currently used.

More particularly, the blocking agent of the invention may be formulated into pharmaceutical compositions that can be used to apply microbicides to effectively prevent transmission of HIV-1 through mucosae, more particularly to prevent the sexual or vaginal transmission of HIV-1. Thus, the compositions are in forms adapted to be applied to the site where sexual intercourse or related intimate contact takes place, such as the genitals, vagina, vulva, cervix, rectum, mouth, hands, lower abdomen, upper thighs, especially the vagina, vulva, cervix, and ano-rectal mucosae.

As appropriate topical compositions there may be cited for example gels, jellies, creams, pastes, emulsions, dispersions, ointments, films, sponges, foams, aerosols, powders, intravaginal rings or other intravaginal drug delivery systems, cervical caps, implants, patches, suppositories or pessaries for rectal, or vaginal application, vaginal or rectal or buccal tablets, mouthwashes.

Particularly, the blocking agents of the invention may be formulated as a gel formulation comprising:
- a topically effective amount of a compound of the present invention;
- a gel-forming compound;
- a buffer;
- a pharmaceutically acceptable diluent, preferably water;
- optionally a humectant; and
- optionally a preservative.

Typical gel formulations can be prepared using natural or synthetic polymers as gelifying agents, and hydrophobic or hydrophilic liquids. Examples of gel-forming compounds commonly employed in gel formulations include polysaccharides which include cellulose derivatives, glycosaminoglycans, gums, starch (a-amylose or amylopectin), and chitosan; carboxyvinylic derivates, vinyl polymers such as polyethylenes, polyehtyelene glycols, e.g. polyethylene glycol 4500, Plastibase® (Plasticized Hydrocarbon Gel), polyacrylic acid, (Carbopols® family, e.g. Carbopol® 940), polymethacrylic acid, polyvinyl pyrrolidone and polyvinyl alcohol; polyacrylamide or polymethacrylamide polymers including clays such as bentonite, Veegum® (R. T Vanderbilt) and Laponite® (Laporte Industries); polyoxyethylene-polyoxypropylene or polyethylene oxides copolymers such as poloxamers, e.g. poloxamer 407, poloxamines; proteins, colloidal silica, soaps, silicones such as dimethylpolysiloxanes or dimeticone, hydrocarbonated bases (mixtures of parafine and vaselines). Useful cellulose derivates include methyl cellulose, hydroxyethyl cellulose, hydroxypropyl cellulose, hydroxypropylmethyl cellulose, carboxymethyl cellulose. Useful glycosaminoglycans include hyaluronic acid, chondroitin, chondroitin-4-sulfate, heparan sulfate and heparin. Useful gums include natural and artificial gums, tragacanth, carrageenan, pectin, agar, alginic acid, dextrans. The glycosaminoglycans may be used to enhance wound healing in combination with any other gel-forming polymer such as, for example, collagen, gelatin, fibronectin. A preferred gelifying agent is hydroxyethyl cellulose, which has additionally bioadhesive properties.

Concentrations of the gel-forming compounds may be varied upon conditions such as the liquid/gel transition temperature, the physical properties sought for the gel and the pH used in the making of the formulations.

Gel forming compounds employed in the present invention are typically water-soluble polymers capable of forming a viscous aqueous solution, or non-water soluble, water-swellable polymers (e.g., collagen) that can also form a viscous solution and that gel upon contact with skin. Gelling agents suitable for use in the present invention should be stable over a wide pH range, especially over the normal acidic pH values found in the vagina.

Buffering agents are used in the gel formulation of this invention to maintain the pH of the vagina within its healthy acidic range (i. e., a pH of less than about 5 and more preferably in the range of about 3.2 to about 4.5) even in the presence of normal amounts of ejaculate. A normal acidic range in the vaginal milieu and environment assists in diminishing the activity of HIV-1 virus. Examples of buffering agents include, without being limited to, lactic acid, phosphoric acid, sodium citrate, sodium hydroxide, sodium phosphate, sodium phosphate dibasic anhydrous, tartaric acid, triethanolamine, citric acid, potassium acid tartrate, benzoic acid, alginic acid, sorbic acid, fumaric acid, ascorbic acid, stearic acid, oleic acid, edetic acid, ethylenediaminetetracetic acid, acetic acid, malic acid, and the like, preferably sodium hydroxide and lactic acid, the latter being additionally a preservative and having certain antimicrobial activity.

The acids may be added as free acids, hydrates, or pharmaceutically acceptable salts. Free acids can be converted to the corresponding salts in situ (i. e., within the vagina). It is generally preferred that several buffering agents are included in the gel of this invention to provide increased buffering capacity. Even more preferably, buffering agents comprise a combination of acid and hydrogen-accepting substance that occur naturally in the human female body that, when applied to the surface of the vagina, maintains the pH level thereon at approximately the pH level of a healthy vagina. Said acid(s) may be selected from the froup consisting of acetic acid, lactic acid, phosphoric acid and sulfuric acid, and combinations thereof. One of the characteristics common to each of said member in said group, is that each acid occurs naturally in the female body. Another common characteristic is each readily contributes to the formation of a buffering system, by temporarily donating hydrogen ion and accepting a cation to form a salt.

Said hydrogen-accepting substance may be selected from the group consisting of potassium hydroxide, sodium hydroxide, calcium hydroxide, potassium carbonate, sodium carbonate and calcium carbonate, and combinations thereof. One of the characteristics common to each of said member in said group, is that each substances is found naturally in the female body. Another common characteristic is each readily contributes to the formation of a buffering system, by temporarily accepting hydrogen ion and donating a cation to form a salt. Such salts may be selected from the group consisting of acetate, lactate, phosphate and sulfate, in combination with said cation from said hydrogen-accepting substance.

The gels of this invention may also include, and preferably do include, humectants. Suitable humectants include, for example, glycerol, polyethylene glycols, propylene glycols, sorbitol, triacetin, and the like. Glycerol, which is the preferred humectant, is a buffer activating component due to its capability of water absortion, or other fluid, from the vaginal environment into the gel. It is believed that such fluid intake prevents the formation of a dry film on the gel when placed within the vagina, providing additional solvent to enhance the application of the gel formulation, or to otherwise enhance its functioning.

The gels of this invention may also include, and preferably do include, a preservative, which amongst other properties, extends the shelf life of the gel formulations. Suitable preservatives include, for example, benzoic acid, sodium benzoate, methyl paraben, ethyl paraben, butyl paraben, propyl paraben, benzylalkonium chloride, phenylmercuric nitrate, chlorhexidine, benzyl alcohol, phenethyl alcohol, propylene glycol, and the like. The preferred preservatives are methyl paraben, and propyl paraben, which both also contribute to the antimicrobial capacity of the gel.

The gels of this invention are prepared using conventional gel preparation techniques. It is desirable, however, to ensure that the buffering agents are solubilized in the final product and that the entrapment of air in the gel is avoided or at least kept to a minimum. To reduce the entrapment of air in the gel, it is generally preferred that the less hydrophilic agents are added in small increments. Alternatively, the gels of this invention can also be prepared in readily dispersable solid forms (e. g., powders, tablets, and the like) which can be converted to the desired gel consistency by action of aqueous based fluids external to or within the vagina when desired. As those skilled in the art will realize, the methods for preparing the gels of this invention can be modified for batch, semi continuous, or continuous operation so long as the resulting gels have the desired and beneficial properties described herein.

The gel formulations can be combined with other active ingredients such as microbicides, antimicrobials, spermicides or other appropriate drugs. If desired, flavorants, scents, fragrances, and colorants can be incorporated into the gel so long as they do not interfere with the protection afforded by the gel. Indeed, incorporation of such flavorants, scents, fragrances, and colorants into the compositions of this invention may provide further protection by increasing the probability that the gel will be used during sexual activity.

The present topical formulations such as the gel formulations described herein are to be used for coating different types of mucosae such as vulvar, vaginal, cervical, ano-rectal, mouth, or skin to prevent the penetration of HIV-1.

The present topical formulations such as the gel formulations described herein could, for example, be applied into the vagina by hand, suppositories, or conventional tampon or syringe techniques. The method of administering or delivering the gel into the vagina is not critical so long as an effective amount of the gel is delivered into the vagina. The present topical formulations such as the gel formulations described herein may also be used for protection during anal intercourse and can be applied using similar techniques.

For vaginal heterosexual intercourse, the present topical formulations such as the gel formulations described herein may be applied into the vagina prior to intercourse. For anal intercourse (heterosexual or homosexual), the present topical formulations such as the gel formulations described herein may be inserted into the rectum prior to intercourse. For either vaginal or anal intercourse, the present topical formulations such as the gel formulations described herein may also act as a lubricant. For added protection it is generally preferred that the present topical formulations such as the gel formulations described herein be applied before intercourse or other sexual activity and that, if appropriate, a condom be used. For even further protection, the present topical formulations such as the gel formulations described herein can be applied as soon as possible after completion of the sexual activity. Although application only after the sexual activity is less recommended, it would still be desirable afterwards if the application was not performed prior to the sexual activity for any reason (e.g., in cases of rape).

The present topical formulations such as the gel formulations described herein are highly suited for the protection of women (as well as their partners) with or without requiring the partner's knowledge of the application of these gels. In addition, reliance on the partner's claim of being concretely HIV-free would not be necessary, neither the agreement to use condoms or other barrier devices for protection.

Intravaginal rings (IVR) are as well suitable drug delivery systems for the vaginal administration of the blocking agents of the invention. IVRs comprise the compound(s) dispersed throughout a biocompatible elastomeric system that forms the delivery device, which preferentially takes the form of a ring. These elastomers preferably include hydrophobic material, such as silicones (organo polysiloxanes including dimethylpolysiloxanes), polyethylene-co-poly (vinyl acetate), styrene-butadiene-styrene block copolymers, polyphosphazenes, poly(isoprene), poly (isobutylene), polybutadienes, polyurethanes, nitrile rubbers, neoprene rubbers or mixtures thereof. Said IVRs can be formulated as sustained-released microbicides, resulting in an extended and stable contact time between the compound and HIV-1 and cells. IVR formulations have already been described in the literature (see WO02076426).

In order to increase the residence time of the topical pharmaceutical composition at the site of administration, it may be advantageous to include a bioadhesive in the different drug delivery systems, in particular a bioadhesive polymer. A bioadhesive may be defined as a material that adheres to a living biological surface such as for example a mucus membrane or skin tissue. The tern bioadhesive is well-known to the person skilled in the art. Thus, the present invention also relates to a pharmaceutical composition comprising a pharmaceutically acceptable carrier and as active ingredient a microbicidal effective amount of a blocking agent of the invention characterized in that the pharmaceutical composition is bioadhesive to the site of application. Preferably, the site of application is the vagina, vulva, cervix, rectum, mouth or skin, most preferred is the vagina and the vulva.

Examples of bioadhesives which may be used in the pharmaceutical compositions of the present invention comprise polyacrylic acid derivatives, such as for example carbopol or polycarbophil, e.g. carbopol 934P, carbopol 940, polycarbophil AA1; cellulose ether derivatives such as for example hydroxypropyl methylcellulose, hydroxypropyl cellulose, hydroxyethyl cellulose, sodium carboxymethyl cellulose, chitosan; natural polymers such as for example alginates, tragacanth, inulin; pregelatinized starch; polysaccharide gums such as xanthan gum, and the like.

Alternatively, formulations of the present invention may be in the form of implants, patches, pads, injections or other preparations for achieving a percutaneous and subcutaneous delivery of the compounds to the cervical, vaginal and rectal tissues.

As already indicated within the gel specifications, the blocking agents of the invention may be used in all the suitable formulations, alone or in combination with other active ingredients, such as antivirals, antibiotics, immunomodulators or vaccines. They may also be used alone or in combination with other prophylactic agents for the prevention of viral infections. The blocking agents of the invention may be used in vaccines and methods for protecting individuals against viral infections over an extended period of time. The compounds may be employed in such vaccines either alone or together with other compounds of this invention or together with other anti-viral agents in a manner consistent with the conventional utilization of reverse transcriptase inhibitors in vaccines. Thus, the blocking agents of the invention may be combined with pharmaceutically acceptable adjuvants conventionally employed in vaccines and administered in prophylactically effective amounts to protect individuals over an extended period of time against HIV-1 infection.

Antiviral compounds which may be used in combination with the blocking agents of the invention may be known antiretroviral compounds such as pentamidine, thymopentin, castanospermine, dextran (dextran sulfate), foscarnet-sodium (trisodium phosphono formate); nucleoside reverse transcriptase inhibitors, e.g. zidovudine (3'-azido-3'-deoxythymidine, AZT), didanosine (2',3'-dideoxyinosine; ddI), zalcitabine (dideoxycytidine, ddC) or lamivudine (2'-3'-dideoxy-3'-thiacytidine, 3TC), stavudine (2',3'-didehydro-3'-deoxythymidine, d4T), abacavir and the like; non-nucleoside reverse transcriptase inhibitors such as nevirapine (11-cyclopropyl-5,11-dihydro-4-methyl-6H-dipyrido-[3,2-b:2',3'-e][1,4]diazepin-6-one), efavirenz, delavirdine, and the like; phosphonate reverse transcriptase inhibitors, e.g. tenofovir and the like; compounds of the TIBO (tetrahydro-imidazo[4,5,1-jk][1,4]-benzodiazepine-2(1H)-one and thione)-type e.g. (S)-8-chloro-4,5,6,7-tetrahydro-5-methyl-6-(3-methyl-2-butenyl)imidazo-[4,5,1-jk][1,4]benzo-diazepine-2(1H)-thione; compounds of the [alpha]-APA ([alpha]-anilino phenyl acetamide) type e.g. [alpha]-[(2-nitrophenyl)amino]-2,6-dichlorobenzene-acetamide and the like; inhibitors of trans-activating proteins, such as TAT-inhibitors, e.g. RO-5-3335, or REV inhibitors, and the like; protease inhibitors e.g. indinavir, ritonavir, saquinavir, lopinavir (ABT-378), nelfinavir, amprenavir, TMC-126, BMS-232632, VX-175 and the like; fusion inhibitors, e.g. T-20, T-1249 and the like; CXCR4 receptor antagonists, e.g. AMD-3100 and the like; inhibitors of the viral integrase; ribonucleotide reductase inhibitors, e.g. hydroxyurea and the like.

Combinations may as well exert a synergistic effect in inhibiting HIV-1 replication when components of the combination act on different or same sites of HIV-1 replication, preferably on different sites. The use of such combinations may reduce the dosage of a given conventional antiretroviral agent which would be required for a desired prophylactic effect as compared to when that agent is administered as a single active ingredient. These combinations reduce potential of resistance to single agent, while minimizing any associated toxicity. These combinations may also increase the efficacy of the conventional agent without increasing the associated toxicity.

Thus, the blocking agents of the invention may also be administered in combination with art-known microbicides, consequently potentiating the prophylactic effect. They can block the infection by creating a barrier between HIV-1 and the site at which transmission will take place, e.g. vulva, vagina; they can kill or immobilize the virus; they can prevent a virus from replicating once it has infected the cells lining the site of transmission, e.g. the cells that line the vaginal wall.

Examples of microbicides may be antibodies: isolated antibodies that counteract HIV-1 are available in the literature. They may be appropriately combined with the blocking agents of the invention to prevent HIV-1 infection.

pH regulators, especially for the vagina may be also suitable. A natural vaginal environment is too acidic for HIV-1 to survive, but semen decreases its acidity, allowing HIV-1 to survive. pH regulators regulate the natural acidity of the vagina making it inhospitable for the HIV. Said regulators encompass the use of Lactobacillus bacteria that produce hydrogen peroxide and thereby help to keep the vaginal environment healthy and acidic. The acidic polymer BufferGel (ReProtect, LLC) is another example of a pH regulator which has in addition spermicidal activity.

Detergents and surfactants may be also suitable. These compounds are able to disrupt the outer shell of viruses and therefore are useful as microbicide and they can be combined with the compounds to prevent HIV-1 infection. Examples of such detergents and surfactants are nonoxynol-9 and octoxynol-9, but all detergents and surfactants that are commonly used in shampoos, toothpastes and cleaning solutions, contact lens solutions may be equally suitable.

Coatings for the pathogen, such as Pro-2000 Gel which contains a synthetic polymer that binds to HIV-1, disrupting the binding of the virus to target cells.

Coatings for the site of transmission, such as for example gels may also suitable. These products may prevent HIV from entering the cells by covering the site of transmission, e.g. the vaginal and vulvar epithelium. Examples, including the gel preparations described above, encompass sulphated and sulphonated polymers such as PC-515 (carrageenan), dextrin 2 sulphate, secretory leukocyte protease inhibitor (SLPI), which binds to the target cells so that they are not accessible to the virus, cyanovirin-N which also binds to the cell, prohibiting cell fusion with HIV.

In the compositions of the present invention, one or more or all of the above-listed microbicides may be combined with a blocking agent of the invention. Thus, the present invention also relates to a pharmaceutical composition comprising a blocking agent of the invention and further comprising one or more components wherein the components are selected from antibiotic peptides, antibodies, pH regulators, detergents or surfactants, coatings for the pathogen, coatings for the site of administration.

One particular example of the combination of microbicides is the combination of blocking agents of the invention with cellulose acetate phthalate (CAP) and/or hydroxypropyl methylcellulose phthalate (HPMCP). CAP and its derivates are excipients which exhibit an additional microbicide effect. CAP formulations have already been described in the literature, EP1030547, U.S. Pat. No. 6,165,493, by Neurath et al.,.

The present invention relates also to a pharmaceutical composition as outlined hereinabove further comprising a spermicidal compound. Said compositions are able to prevent at the same time conception and HIV-1 infection. Suitable spermicides are for example nonoxynol-9, octoxynol-9, menfegol, benzalkonium chloride, N-docasanol.

Those of skill in the prophylaxis of HIV-1 infection could determine the microbicidal effective amount from the test results presented here and may range from about 1 ng to about 10 mg, in particular from about 10 ng to about 1 mg, more in particular from about 100 ng to about 100 [mu]g and preferably from about 500 ng to about 50 µg of active ingredient per application or unit dose, in particular an application or unit dose of an immediate release formulation.

It may be appropriate to apply the required dose as unit dosage forms. The volume of a unit dose, in particular the unit dose of an immediate release formulation, whether or not in a unit dosage form, may range in the case of a topical formulation from about 10µml up to about 25 ml of topical formulation and in particular from about 1 ml up to about 10 ml of topical formulation. In the case of a gel or a cream for instance, a convenient unit dose could range between about 1 ml and about 5 ml. For instance in the case of topical formulations, in particular topical formulations for immediate release, as mentioned herein, e.g. a gel, a cream and the like, the active ingredient may be present in a concentration ranging from about 1 nM up to about 10 mM, in particular from about 10 nM up to about 1 mM, more in particular from about 100 nM up to about 100 µM and preferably from about 1 µM to about 100 µM.

It is evident that said effective amount may be lowered or increased depending on the particular compound being used, on the response of the treated subject and/or depending on the evaluation of the physician prescribing the compounds of the instant invention.

The invention will be further illustrated by the following figures and examples. However, these examples and figures should not be interpreted in any way as limiting the scope of the present invention.

### FIGURES:

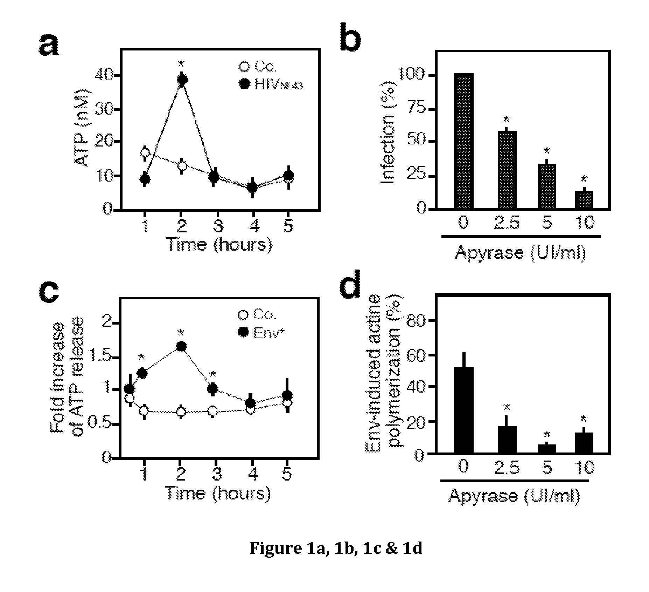

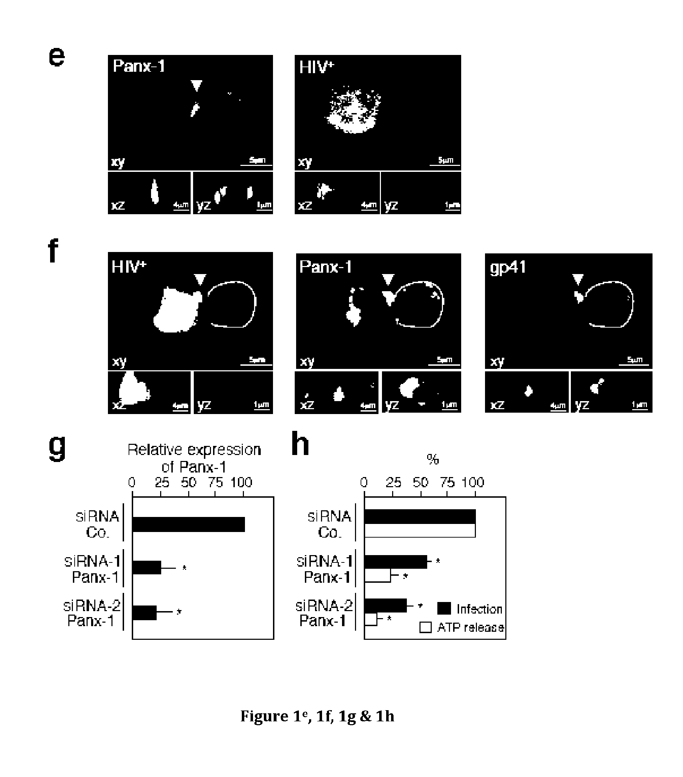

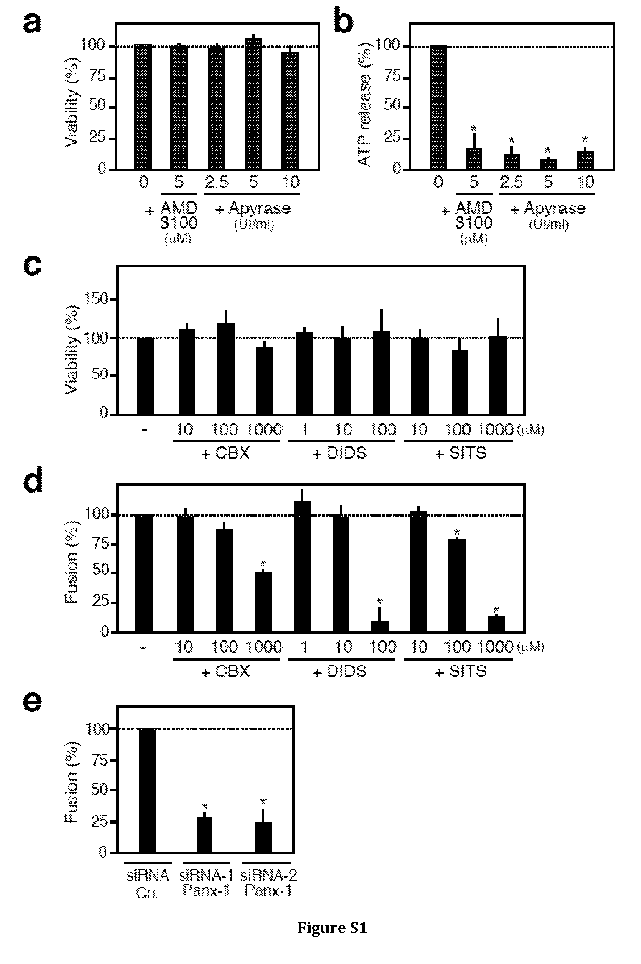

**Figure 1** ATP release through pannexin-1 hemichannels modulates interactions of HIV-1 with target cells. (a) ATP released during HIV-1 infection (black circle) or in absence of infection (white circle) was determined at different time points by ATP-dependent bioluminescence in 3 independent experiments. One representative experiment is shown (mean ± SEM; **P* < 0.05). (**b**) Effect of apyrase on HIV-1 infection. CD4⁺CXCR4⁺ cells were infected with HIV_{NL43} (multiplicity of infection = 1) during 3 hours in presence of different concentrations of apyrase. Then, the medium containing apyrase was removed, replaced by complete medium, and the target cell infectivity was determined 24 hours after infection using CD4⁺CXCR4⁺ cells that contain a Tat-inducible β-galactosidase (β-Gal) reporter gene (means ± SEM; n = 3; **P* < 0.05). (c) ATP released during the coculture of Env-expressing cells with HIV-1 target cells (black circle) was also evaluated at different time points. ATP release was expressed as a fold increase of ATP release during coculture of Env-expressing cells with HIV-1 target cells (versus coculture of cells that do not express Env (white circle) with target cells). Error bars indicate SD of 3 independent determinations (means ± SD; **P* < 0.05). (**d**) Effect of ATP depletion by apyrase on HIV-1 envelope mediated actin polymerization. Actin polymerization between two interacting cells was determined in 3 independent experiments (means ± SEM; **P* < 0.05) by fluorescent microscopy (**e**) Localization of pannexin-1 at the contact site between primary HIV-1-infected lymphoblasts (green) and primary uninfected lymphoblasts, visualized by confocal microscopy. The arrow head points to host cell pannexin-1 accumulation on the interface observed between primary infected and uninfected lymphoblasts. A representative micrograph is shown. Merge images of *xz* and *yz* optical sections are also shown. (**f**) Accumulation of pannexin-1 on the virological synapse mediated by HIV-1 envelope is revealed by the detection of pannexin-1 and the HIV-1 envelope glycoprotein gp41 at the contact site between primary HIV-1-infected lymphoblasts (blue) and primary uninfected lymphoblasts. The images in (e,f) are representative of at least of 40 cells in 3 independent experiments. Note the circular distribution of pannexin-1 on the virological synapse. (g) Pannexin-1 mRNA expression in cells transfected with a small interfering RNA targeting pannexin-1 was estimated by quantitative RT-PCR analysis in 3 independent experiments (means ± SEM; **P* < 0.05). (h) Effect of pannexin-1 knockdown on ATP release and on host cell infectivity. CD4⁺CXCR4⁺ cells were transfected during 48 hours with siRNAs specific for pannexin-1 and then infected with HIV_{NL43} (multiplicity of infection = 1). Th effects of pannexin-1 depletion on ATP release and on host cell infectivity were determined respectively after 3 hours (by ATP-dependent bioluminescence) and after 24 hours using CD4⁺CXCR4⁺ cells that contain a Tat-inducible β-galactosidase β-Gal) reporter gene). Results are means ± SEM; n = 3; **P* < 0.05.

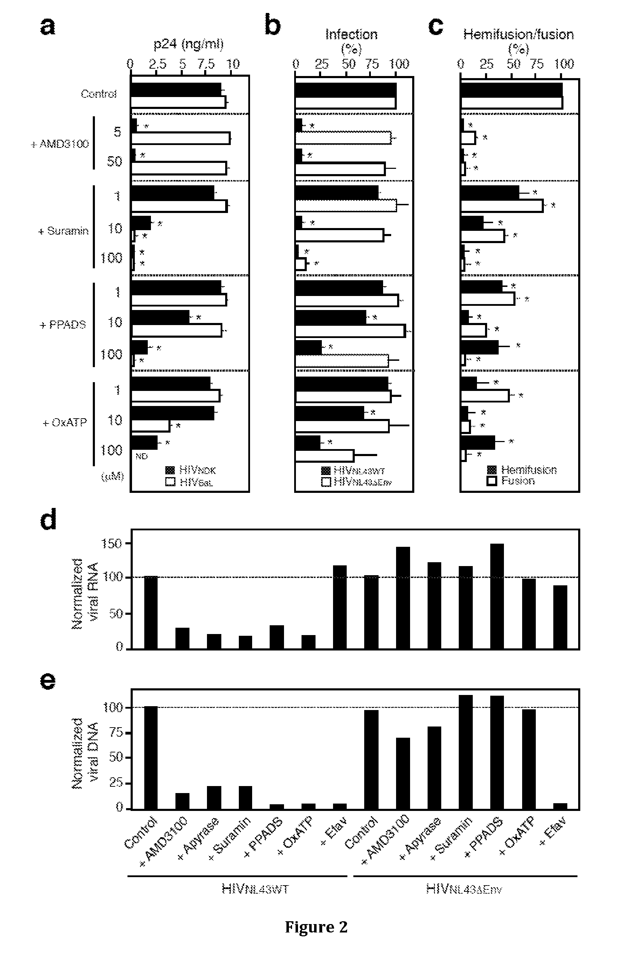

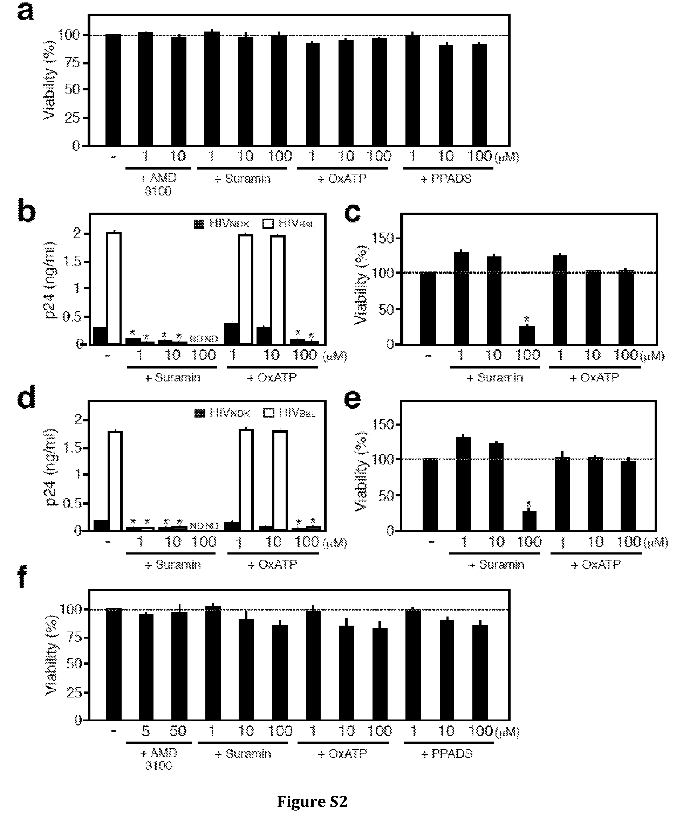

**Figure 2** Purinergic receptors modulate HIV-1 infection. **(a, b, c)** Effects of the general P2-receptor antagonist suramin, pyridoxal-phosphate-6-azophenyl-2',4'-disulfonate (PPADS) and OxATP on viral yield (a), host cell infectivity (b) and hemifusion/fusion (c) observed during infection with HIV_{NDK}, HIV_{BaL}, HIV_{NL43WT}, HIV_{NL43ΔEnv} (a, b) or during coculture of Env-expressing cells with HIV-1 target cells (c) Columns in **a-c** show means ± SEM (n = 3; **P* < 0.05). **(d, e)** Effects of AMD3100, apyrase, suramin, PPADS, OxATP and Efavirenz on viral RNA expression and on total viral DNA expression of wild-type (HIV_{NI43WT}) or VSVG pseudotyped HIV-1 (HIV_{NL43AEnv}). Viral RNA and Total viral DNA were estimated from CD4⁺CXCR4⁺ cells that were infected with HIV_{NL43WT} or pseudotyped HIV_{NL43ΔEnv} virus in the absence or presence of 5 µM AMD3100, 10 UI/ml apyrase, 10 µM suramin, 100 µM PPADS, 100 µM OxATP or 500 nM Efavirenz (Efav). Viral RNA was detected by quantitative PCR as described in Methods. Note that internalization of pseudotyped HIV_{NL43ΔEnv} virus is not reduced by these inhibitors (d). Total viral DNA was determined using PCR quantification (as described in Methods) and results are shown as a normalized ratio of HIV-1 DNA *versus* GAPDH control DNA. The results are representative for 3 independent experiments.

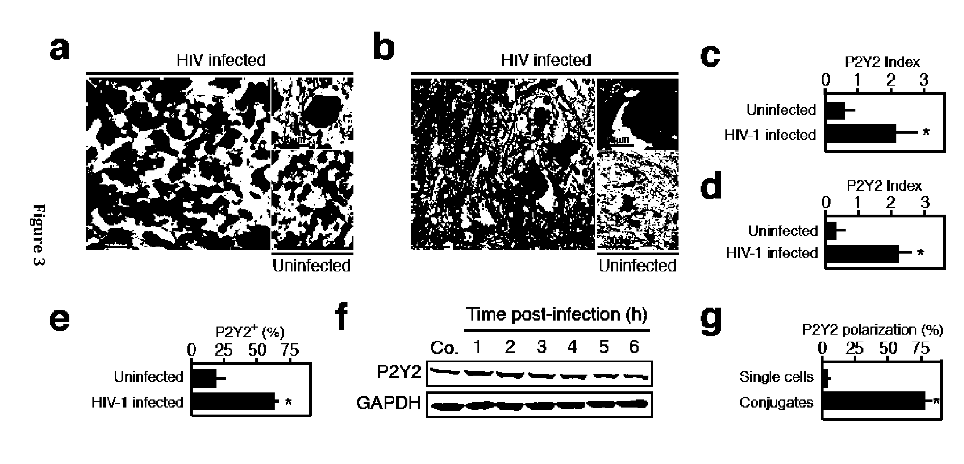

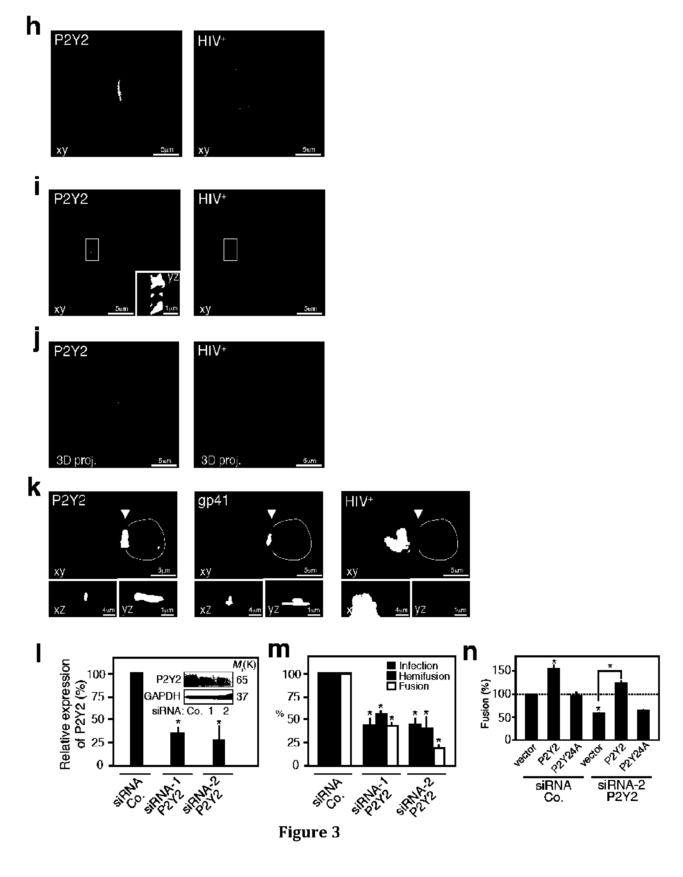

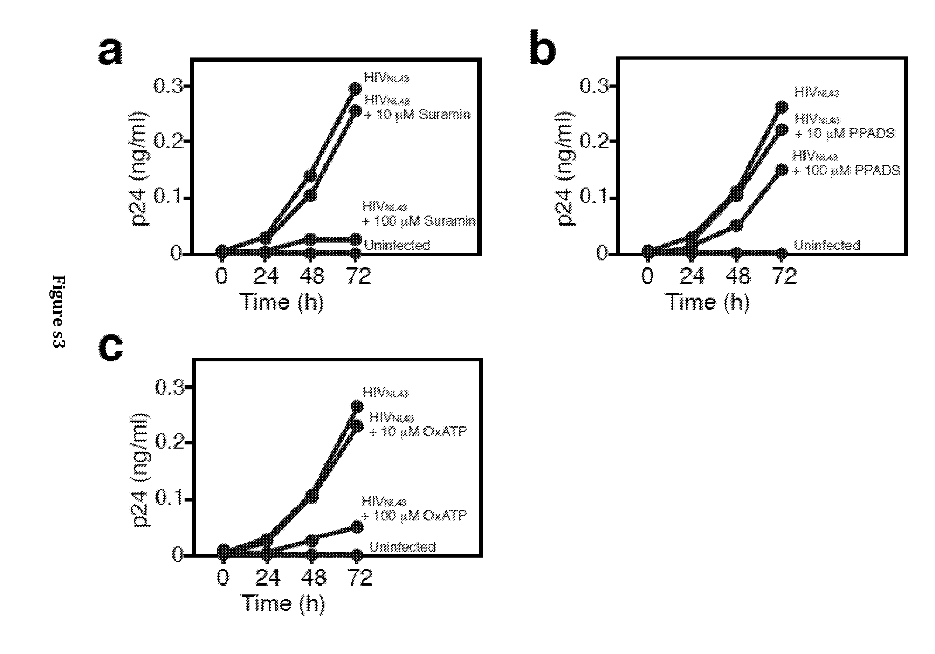

**Figure 3** Role of P2Y2 on HIV-1 infection. **(a, b)** Representative immunohistochemistries of P2Y2 expression on lymph node biopsies (a) and on frontal cortex biopsies (b) obtained from HIV-1 infected patients. The upper insert shows a positive fused cell and the lower insert illustrates the absence of staining of lymph node biopsies from uninfected patients. **(c, d)** P2Y2 expression in lymph node (c) and frontal cortex biopsies (d) was determined and revealed by the P2Y2 index as described in Methods. The error bars represent means ± SEM (n = 3; **P* < 0.05). **(e)** Detection by flow cytometry of P2Y2 expression on peripheral blood mononucleated cells obtained from uninfected or untreated HIV-1 infected patients (means ± SEM; n = 3; **P* < 0.05) . **(f)** Kinetics of P2Y2 expression during infection with HIV-1. CD4⁺CXCR4⁺ cells were infected with HIV_{NL43} (multiplicity of infection = 1) during the indicated times. Then P2Y2 expression was evaluated by immunoblot. Representative immunoblots of 3 independent experiments are shown. **(g)** Frequency of P2Y2 polarization at the virological synapse induced by HIV-1 between lymphoblasts. Data in g are means ± SEM of 3 independent experiments. Asterisk indicates P < 0.05. **(h, i, j)** Detection of P2Y2 subcellular localization during interaction between HIV-1 infected lymphoblasts and uninfected lymphoblasts, visualized by confocal microscopy. Note P2Y2 accumulation at the virological synapse induced by HIV-1 (h, i, j). Confocal microscopy shows P2Y2 polarization between HIV-1 infected lymphoblasts and uninfected lymphoblasts (h). The insert in **i** represents a *yz* optical section and shows the circular distribution of P2Y2. Contrast phase in **h and i** revealed interacting lymphoblasts. 3D projection reveals a ring like distribution of P2Y2 between interacting lymphoblasts (j). **(k)** Accumulation of P2Y2 on virological synapse mediated by HIV-1 envelope between interacting lymphoblasts. Note the ring like distribution of P2Y2 around the HIV-1 envelope glycoprotein gp41 between HIV-1 infected lymphoblasts and uninfected lymphoblasts. Images in **h-k** are representative at least of 4 independent experiments. **(1)** Knockdowns of P2Y2 were confirmed using two distinct siRNA by quantitative RT-PCR analysis (as described in Methods) and by Western blot. **(m)** Effects of P2Y2 knockdowns on HIV_{NL43WT} infection, hemifusion, and fusion induced by HIV-1 envelope. **(n)** Effect of P2Y2 overexpression on HIV-1-envelope induced fusion. Cells depleted or not of P2Y2 were transfected with human P2Y2 wild-type or human P2Y2/4A mutant. Cell fusion mediated by the HIV-1 envelope was then evaluated by determining Tat-inducible ß-galactosidase (ß-gal) activity during coculture of HIV-1 Env-expressing cells with CD4⁺CXCR4⁺ expressing cells, as described in Methods. Error bars indicate SEM of 3 independent determinations for (1), (m) and (n).

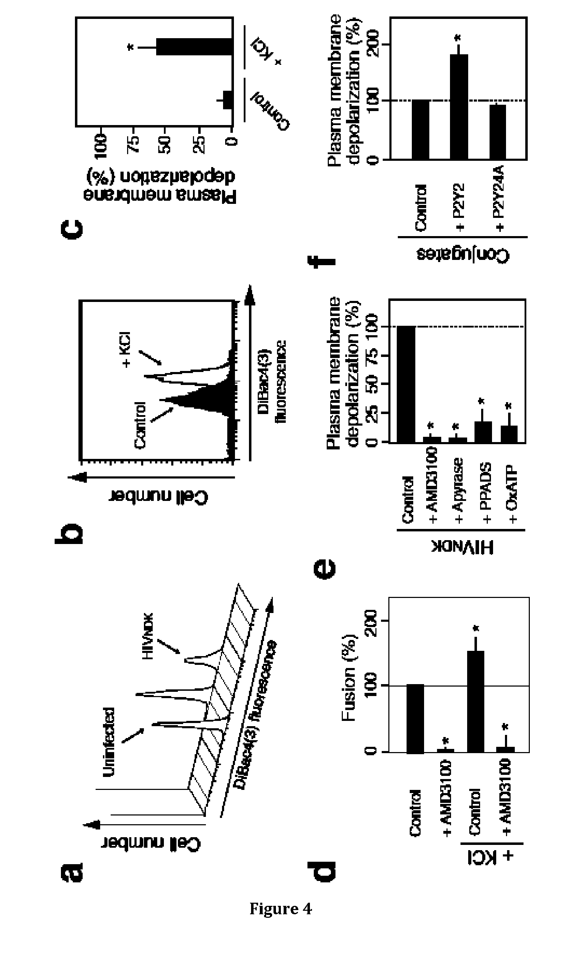

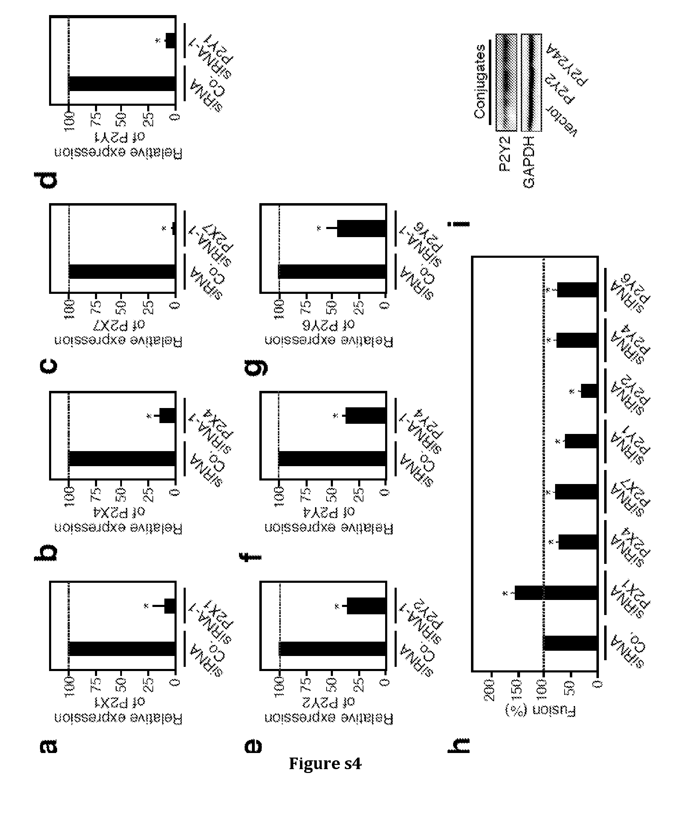

**Figure 4** P2Y2 controls plasma membrane depolarization during HIV-1 Env-mediated fusion.. **(a)** Plasma membrane depolarization of primary lymphoblasts after 1 hour of infection with HIV_{NDK} (multiplicity of infection (MOI) of 1). Plasma membrane depolarization was determined by evaluating the increase of fluorescence of DiBac4(3) probe by flow cytometry. Representative profiles are shown. **(b)** Representative profiles of plasma membrane depolarization of CD4⁺CXCR4⁺ cells treated with 70 mM KCl for 3 hours. Plasma membrane depolarization was determined by evaluating the increase of fluorescence of the DiBac₄(3) probe by flow cytometry. **(c)** Frequency of CD4⁺CXCR4⁺ cells showing plasma membrane depolarization after 3 hours of 70 mM KCl treatment (means ± SEM; n = 3; **P* < 0.05). **(d)** Effects of 5 µM AMD3100 on HIV-1 Env-induced fusion realized in the absence or presence of 70 mM KCl. Cell fusion was determined as described in Methods. The error bars represent means ± SEM (n = 3; **P* < 0.05). **(e)** Effects of AMD3100, apyrase, PPADS and OxATP on plasma membrane depolarization after 1 hour of HIV_{NDK} infection. Data shown were determined in 3 independent experiments (means ± SEM; **P* < 0.05) using the DiBac₄(3) probe and flow cytometry. **(f)** P2Y2 expression modulates plasma membrane depolarization induced by HIV-1 envelope. Effects of transfection of human P2Y2 wild-type or mutant (P2Y2/4A) constructs on HIV-1 envelope induced plasma membrane depolarization were determined as described in **a** (means ± SEM; n = 3; **P* < 0.05).

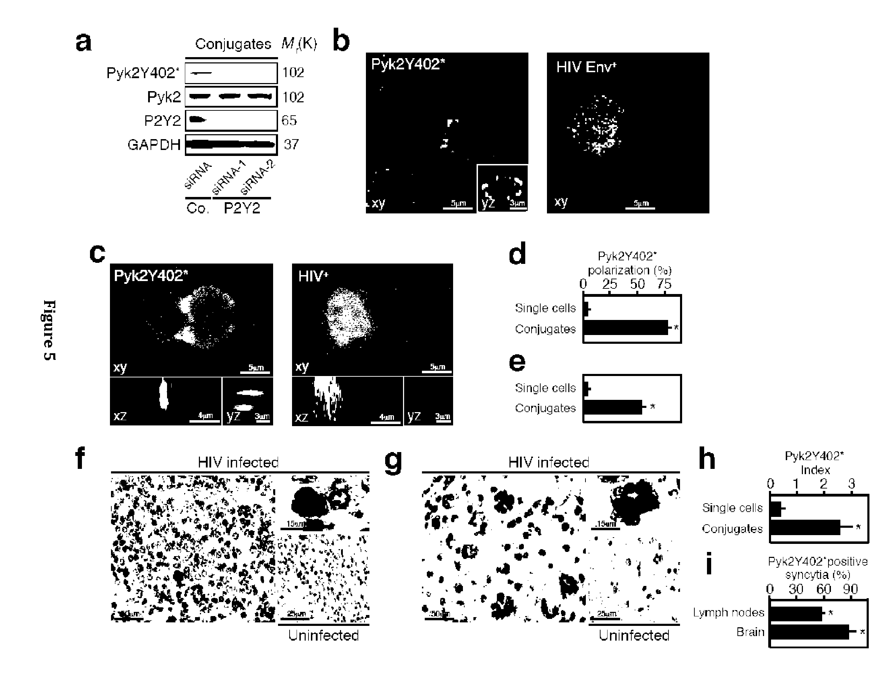

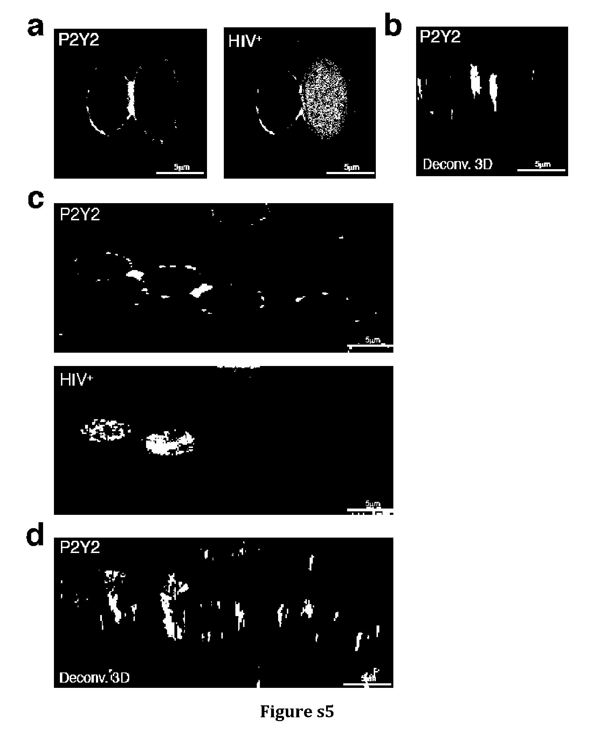

**Figure 5** P2Y2 induces Pyk2 activation during HIV-1 infection. **(a)** P2Y2 depletion inhibits Pyk2Y402* phosphorylation induced during HIV-1 Env-mediated conjugate formation. Effects of P2Y2 knockdown on Pyk2Y402* and on Pyk2 expression were determined by immunoblot as described in Methods. The blot is representative for 3 independent experiments. **(b)** Confocal microscopy reveals Pyk2Y402* polarization at the contact site between HIV Env-expressing cells and CD4⁺CXCR4⁺ cells. Representative Pyk2Y402* polarization between HIV Env⁺ cells and CD4⁺CXCR4⁺ cells is shown. Note the ring-like distribution of Pyk2Y402* between interacting cells. The insert in **b** shows the *yz* section. **(c)** Detection of Pyk2Y402* polarization during interaction between HIV-1 infected lymphoblasts and uninfected lymphoblasts by confocal microscopy. Note the accumulation of Pyk2Y402* on the target cell during virological synapse formation induced by HIV-1. The left insert represents a *xz* optical section and polarization of Pyk2Y402* between interacting cells. The right insert reveals a *yz* optical section and shows peripheral distribution of Pyk2Y402* in the contact site within conjugates. Images are representative of 4 independent experiments. **(d, e)** Frequency of Pyk2Y402* polarization between single cells and conjugates during coculture of HIV Env⁺ cells and CD4⁺CXCR4⁺ (d) or coculture of HIV-1 infected lymphoblasts and uninfected lymphoblasts (e) was determined in 3 independent experiments (means ± SEM; **P* < 0.05). **(f, g)** Detection of Pyk2Y402* on lymph node sections (f) and on frontal cortex biopsies (g) obtained from HIV-1-infected patients. Note the positive staining of fused cells observed on lymph nodes (f) and on frontal cortex (g) biopsies obtained from HIV-infected patients (on the upstream insert) and the negative staining pattern of lymph node (f) and of frontal cortex (g) obtained from uninfected patients. (**j**) Positive correlation between Pyk2Y402* detected on peripheral bood mononucleated cells and viremia obtained from untreated HIV-1 carriers. The p value corresponds to the correlation coefficient. **(k)** Effect of highly active antiretroviral therapy (HAART) on Pyk2Y402* detected on peripheral blood mononucleated cells obtained from treated patients, as compared to untreated HIV-1 infected patients (Student's t test, **P* = 0.0009). **(1)** Effects of P2Y2 knockdown on Pyk2Y402* polarization at the contact site between conjugates. **(m)** Knockdown of Pyk2 with two distinct siRNAs was confirmed using by immunoblot. The blot is representative of three independent determinations. **(n)** Effects of Pyk2 knockdowns on HIV_{NL43WT} infection and hemifusion/fusion mediated by the HIV-1 envelope (means ± SEM; n = 3; **P* < 0.05).

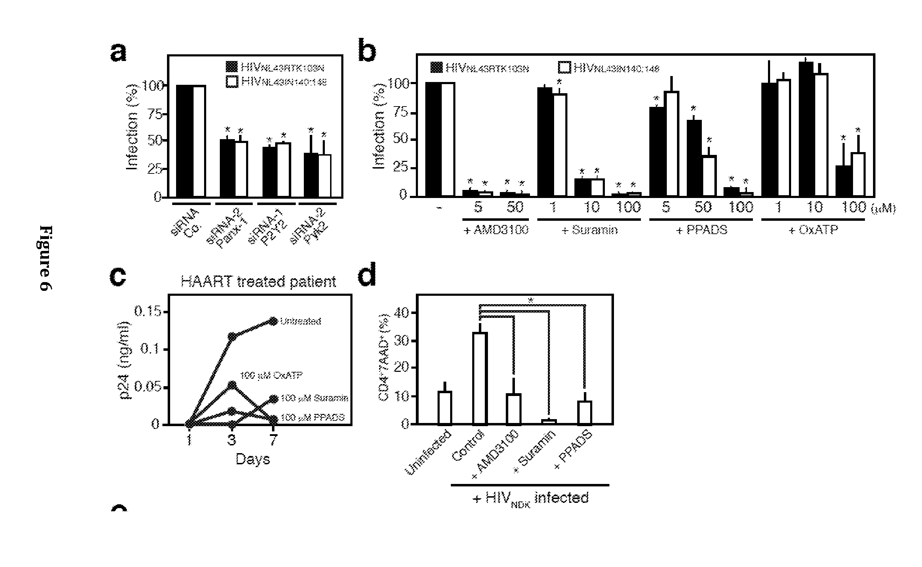

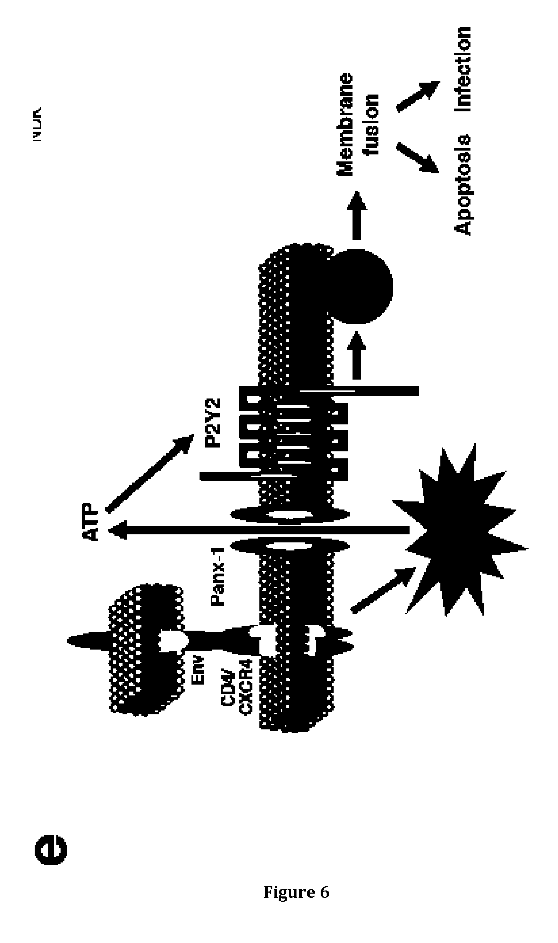

**Figure 6** Inhibition of Pannexin-1, P2Y2 and Pyk2 reduces HIV-1 infectivity and T cell depletion associated with HIV-1 infection. **(a)** Effects of Pannexin-1, P2Y2 and Pyk2 knockdowns on the infectious activity of therapy-resistant HIV-1 mutated viruses (HIV_{NL43RTK103N} and HIV_{NL43IN140:148}). Target cell infectivity was determined using CD4⁺CXCR4⁺ cells that contain a Tat-inducible ß-galactosidase (ß-Gal) reporter system as described in Methods. **(b)** Effects of different concentrations of suramin, PPADS and OxATP on HIV mutant infectivity, as described in a. (c) Treatment of HIV target cells with suramin, PPADS and OxATP reduces viral propagation triggered during ex vivo stimulation of peripheral blood mononucleated cells obtained from HAART-treated patients with undetectable viremia. The effects of the inhibitors on viral propagation were determined after 1, 3 and 7 days of infection, as described in Methods. Results shown are representative of those obtained from three HAART-treated patients. **(d)** Treatment of HIV_{NDK} infected (PHA/IL-2 stimulated) lymphoblasts with suramin and PPADS reduces viral infection and CD4⁺ T cell depletion. Infection (not shown) and CD4⁺ T cell depletion were analyzed by flow cytometry by determining intracellular p24 antigen staining and 7-Amino-actinomycin D (7-AAD) uptake, as described⁶³. **(e)** Involvement of pannexin-1, ATP release, P2Y2 activation and Pyk2Y402* phosphorylation in early stages of HIV-1 infection. Binding of HIV-1 envelope to cellular coreceptors leads to rapid ATP release from host cells through pannexin-1, which activates purinergic receptor P2Y2, leading to activation (phosphorylation) of Pyk2 (Pyk2Y402*). P2Y2 and Pyk2 control plasma membrane depolarization, hemifusion and fusion, which are required for HIV-1 infection.
**Supplementary** **Figure 1** Pannexin-1-dependent ATP release during HIV-1 infection and during HIV-1 Env-mediated fusion. **(a)** Effect of apyrase and AMD3100 on target cell viability. The viability of cells incubated for 3 hours with different concentrations of apyrase or AMD3100 (as indicated) and then infected with HIV-1NL43 for 2 days, was assessed by the MTT (3-[4,5-dimethylthiazol-2-yl]-2,5-diphenyl tetrazolium bromide) assay, as detailed in Methods. **(b)** Effects of apyrase and AMD3100 on ATP release detected during HIV-1 infection. CD4⁺CXCR4⁺ cells were infected with HIV_{NL43} (multiplicity of infection = 1) in absence or in presence of 2.5, 5 or 10 UI/ml apyrase or 5 □M AMD3100. After 2 hours of infection, ATP release was determined as described in Methods. **(c, d)** Effects of pharmacological inhibitors of Pannexin-1, carbenoxolone (CBX), disodium 4,4-diisothiocyanatostilbene-2,2-disulfonate (DIDS) and disodium 4-acetamido-4-isothiocyanato-stilben-2,2-disulfonate (SITS) on cell viability (c) and on HIV-1 envelope-mediated fusion (d). Cell viability in **c** was assessed as in **a** (mean ± s.e.m; n = 3; **P* < 0.05). Fusion between HIV-1 Env-expressing cells (that also express Tat protein) and CD4⁺CXCR4⁺ target cells (that contain Tat-inducible ß-galactosidase) was determined by measuring ß-galactosidase (ß-Gal) activity (d), as described in Methods. **(e)** Effects of Pannexin-1 knockdowns on HIV-1 Env-mediated fusion, as described in (c). Data in (a), (b), (c), (d) and (e) were determined in 3 independent experiments (mean ± s.e.m; **P* < 0.05).
**Supplementary** **Figure 2** Impact of P2 inhibitors on cell viability, viral production and host cell infectivity during HIV infection. (**a** - **e)** Primary human lymphoblasts (a), human monocyte-derived macrophages (b, c), human monocyte-derived dendritic cells (d, e) and human CD4⁺CXCR4⁺ target cells (f) were infected with X4 tropic (HIV-1_{NDK} or HIV_{NL43}) or R5 tropic (HIV-1_{BaL}) HIV-1 strains (multiplicity of infection (MOI) of 1) for 3 days in the presence or absence of the indicated concentrations of the P2-receptor antagonist suramin, the P2X-selective antagonist oxidized ATP (OxATP) or the P2Y-selective antagonist pyridoxal-phosphate-6-azophenyl-2',4'-disulfonate (PPADS). Cell viability was determined after 3 days of infection. The effects of these inhibitors on viral production were determined after 3 days of infection, as previously described ⁶⁴. Results were obtained from 3 independent experiments (mean ± s.e.m; **P* < 0.05).
**Supplementary** **Figure 3** Effects of AMD3100, suramin, PPADS and OxATP on viral yield during HIV-1 infection. **(a, b, c)** Effects of P2 inhibitors on viral yield during infection of the C8166 T cell line with clinical HIV-1 isolates. Kinetics of HIV-1 virion release during C8166 T cell line infection in the absence or presence of the indicated concentrations of the P2-receptor antagonist suramin, the P2Y-selective antagonist pyridoxal-phosphate-6-azophenyl-2',4'-disulfonate (PPADS) or the P2X-selective antagonist oxidized ATP (OxATP) were determined. HIV-1 viral yield was evaluated by quantifying p24 antigen concentrations in the medium. The results are representative for 3 independent experiments.
**Supplementary** **Figure 4** P2Y2 controls HIV-1 Env mediated fusion. (**a**-**g**) P2X1-, P2X4-, P2X7-, P2Y1-, P2Y2-, P2Y4-, P2Y6- mRNA expression in CD4⁺CXCR4⁺ cells transfected with siRNA targeting P2X1, P2X4, P2X7, P2Y1, P2Y2, P2Y4 or P2Y6 were estimated with quantitative RT-PCR analysis. Error bars indicate SEM of duplicate determinations. **(h)** Effects of P2X1-, P2X4-, P2X7-, P2Y1-, P2Y2-, P2Y4- and P2Y6-depletion on HIV-1 Env-induced fusion. The fusion assay was performed as in Fig. S1d. Note that P2Y2 depletion reduces efficiently HIV-1 Env-induced fusion. Error bars in (h) represent SEM of triplicate determinations and asterisk indicates P < 0.05. (i) Transfection of human P2Y2 wild-type and human P2Y2/4A mutant construct in target cells was confirmed by Western blot. The blots are representative of three independent experiments.
**Supplementary** **Figure 5** Detection of polarized P2Y2 at the virological synapse mediated by HIV_{NDK}. (**a**, **c)** Fluorescent microscopy shows P2Y2 polarization between HIV-1 infected lymphoblasts and uninfected lymphoblasts (a, c), but also between HIV-1 infected lymphoblasts (in green) (c). **(b, d)** 3D reconstruction of merged images also reveals a similar distribution of P2Y2 between uninfected and HIV-1-infected lymphoblasts. Images are representative of three independent experiments.

### EXAMPLE:

### Methods :

**Antibodies and reagents.** Polyclonal rabbit antibodies for the detection of P2Y2 were purchased from Abcam or Alomone labs. Polyclonal rabbit antibodies against Pannexin-1 were obtained from Abcam and Millipore. The polyclonal rabbit antibodies used for the detection of the phosphorylated form of Pyk2 on tyrosine 402 (Pyk2Y402*) were from Cell Signaling Technology. Suramin, pyridoxal-phosphate-6-azophenyl-2',4'-disulfonate (PPADS), oxidized ATP (OxATP), carbenoxolone (CBX), disodium 4,4-diisothiocyanatostilbene-2,2-disulfonate (DIDS), disodium 4-acetamido-4-isothiocyanato-stilben-2,2-disulfonate (SITS), apyrase, AMD3100 and phytohemagglutinin-P (PHA) were purchased from Sigma-Aldrich. Human recombinant macrophage-colony stimulating factor (rhM-CSF), granulocyte-colony stimulating factor (rhGM-CSF), interleukin-4 (rhIL-4) and interleukin-2 (rhIL-2) were obtained from Peprotech. HeLa cells stably transfected with the Env gene of HIV-1 LAI/IIIB (HeLa Env) and HeLa cells transfected with CD4 (HeLa CD4) were cultured alone or together at a 1:1 ratio in Dulbecco's modified Eagle's medium supplemented with 10% FCS, 2 mM L-glutamine and penicillin/streptomycin (Invitrogen) in the absence or in presence of tested molecules.

**HIV-1 infection and purinergic inhibitors.** Primary human lymphoblasts, monocyte-derived macrophages, monocyte-derived dendritic cells or CD4⁺CXCR4⁺ target cells were seeded into 96-well culture plates (5 x 10⁵ cells /well) and incubated 30 minutes with increasing concentrations of CXCR4 antagonist (AMD3100), the P2-receptor antagonist suramin, the P2X-selective antagonist oxidized ATP (OxATP) or the P2Y-selective antagonist pyridoxal-phosphate-6-azophenyl-2',4'-disulfonate (PPADS). Then, cells were incubated with wild-type HIV-1, VSV-pseudotyped HIV-1, therapy-resistant HIV-1 mutated viruses (1 ng of p24 antigen) or clinical HIV-1 isolates (15 ng of p24 antigen) for 3 hours at 37°C in a 5% CO₂ atmosphere. After washing out unabsorbed virus, cells were cultured for the indicated times in the presence of tested molecules. ATP release, plasma membrane depolarization, virus-cell fusion were determined as described in Methods. Viral RNA and DNA, viral production, cell viability and cell death were analyzed, as previously described ^{63,} 64

**RNA interference.** Small interfering RNAs specific for Pannexin-1 (siRNA-1: 5'-GCUCCAAGGUAUGAACAUA-3' SEQ ID NO:2), P2X1 (siRNA-1: 5'-GCACUGCAGACCCAUCUAU SEQ ID NO:3, siRNA-2: 5'-GUGGCAGUCAGUAACCAUA-3' SEQ ID NO:4), P2X4 (siRNA-1: 5'-GGAAAACUCCCUCUUCGUC-3 SEQ ID NO:5', siRNA 2: ON-TARGET plus SMART pool P2X4), P2X7 (siRNA-1: 5'-GCUGUCGCUCCCAUAUUUA-3' SEQ ID NO:6, siRNA-2: ON-TARGET plus SMART pool P2X7), P2Y1(siRNA-1: 5'-GGUCUCAUUAUAUAUUGGG-3' SEQ ID NO:7, siRNA-2: 5'-AUUAUCACCAGGUAAAUGGAUUUCC-3' SEQ ID NO:8), P2Y2 (siRNA-1: 5'-GGAUAGAAGAUGUGUUGGG-3' SEQ ID NO:9, siRNA-2: 5'-GGCUGUAACUUAUACUAAA-3' SEQ ID NO:10), P2Y4 (siRNA-1: 5'-GGACAGUAGCUGCUCUACU-3' SEQ ID NO:11, siRNA-2: 5'-GAAAAGAGGAAGAAGCACC-3' SEQ ID NO:12), P2Y6 (siRNA-1: 5'-GAACUUCAAGCAACUGCUG-3' SEQ ID NO:13, siRNA-2: 5'-GGUGCUCACAAAAAUACA-3' SEQ ID NO:14), P2Y12 (siRNA-1: CUGUACCGGUCAUACGUAA-3 SEQ ID NO:15'), PYK2 (siRNA-1: 5'-GCUGAUCGGCAUCAUUGAA-3' SEQ ID NO:16, siRNA-2: 5'-GGCGGUUCUUCAAGGAUAU-3' SEQ ID NO:17) and control sirRNA (5'-GCCGGUAUGCCGGUUAAGU-3' SEQ ID NO:18) were transfected 48 hours before cell fusion using Oligofectamine (Invitrogen), according to manufacturer's instructions. Silencing of purinergic receptors was determined by quantitative PCR using the following probes: P2X₁: 5'-GCTGGTGCGTAATAAGAAGGTG-3' (SEQ ID NO:19), 5'-ATGAGGCCGCTCGAGGTCTG-3' (SEQ ID NO:20) ; P2X₄: 5'-GATACCAGCTCAGGAGGAAAAC-3' (SEQ ID NO:21) ,5'-GCATCATAAATGCACGACTTGAG-3' (SEQ ID NO:22); P2X₇: 5'-TGATAAAAGTCTTCGGGATCCGT-3' (SEQ ID NO: 23) ,5'TGGACAAATCTGTGAAGTCCATC-3' (SEQ ID NO:24) ; P2Y₁: 5'-CTTGGTGCTGATTCTGGGCTG-3' (SEQ ID NO:25) , 5'-GCTCGGGAGAGTCTCCTTCTG-3' (SEQ ID NO:26) ; P2Y₂: 5'-CCGCTCGCTGGACCTCAGCTG-3' (SEQ ID NO:27) , 5'-CTCACTGCTGCCCAACACATC-3' (SEQ ID NO:28) ; P2Y: 5'-CGCTGCCCACCCTCATCTAC-3' (SEQ ID NO:29), 5'-CCGAGTGGTGTCATGGCACAG-3' (SEQ ID NO:30); P2Y₆: 5'-AACCTTGCTCTGGCTGACCTG-3' (SEQ ID NO:31) , 5'-GCAGGCACTGGGTTGTCACG-3' (SEQ ID NO:32); P2Y₁₂: 5'-ACCGGTCATACGTAAGAACGAG-3' (SEQ ID NO:33) ,5'-GCAGAATTGGGGCACTTCAGC-3' (SEQ ID NO:34) ;GAPDH :5'-CAATGCATCCTGCACCACCAA-3' (SEQ ID NO:35) , '5-GTCATTGAGAGCAATGCCAGC-3' (SEQ ID NO:36).

**Immunoblots and immunofluorescence.** Total cellular proteins were extracted in 250 mM NaCl-containing lysis buffer (250 mM NaCl, 0.1% NP40, 5 mM EDTA 10 mM Na₃VO₄, 10 mM NaF, 5mM DTT, 3 mM Na₄P₂O₇ and the protease inhibitor cocktail (EDTA-free protease inhibitor tablets, Roche). Protein extracts (50 µg) were run on 4-12% SDS-PAGE and transferred at 4°C onto a nitrocellulose membrane. After blocking, membranes were incubated with primary antibody at room temperature overnight. Horse radish peroxidase-conjugated goat anti-mouse or anti-rabbit (Southern Biotechnology) antibodies were then incubated during 1 h and revealed with the enhanced ECL detection system. After 3 days of infection with HIV_{NDK} (multiplicity of infection of 1), PHA/IL-2 stimulated peripheral blood lymphocytes were stained with 10 µM of 5-chloromethylfluorescein diacetate (Cell Tracker Green CMFDA, Invitrogen) and cocultured with uninfected lymphoblasts for 48 hours. Cells were then fixed in 4% paraformaldehyde/ phoshate-buffered saline (PBS) for 30 minutes, permeabilized in 0.1% SDS in PBS and incubated with FCS for 20 minutes, as previously described ⁶⁵. Indicated antisera were used for immunodetection in PBS containing 1 mg/ml BSA and revealed with goat anti-rabbit IgG conjugated to Alexa 546 (red) fluorochromes from Invitrogen. Cells were analyzed by fluorescent confocal microscopy on a Zeiss LSM510 using a 63x objective. Z series of optical sections at 0.2-µm increments were acquired. After image deconvolution, three-dimensional image reconstructions of interacting cells were performed using the IsoSurface function of Imaris 5.7 Software (Bitplane AG).

**Viral and pseudo-viral constructs.** Viral stocks of wild-type X4, R5 and resistant HIV-1 were obtained after transfection of 293T with virus encoding plasmids as previously described ^{43, 66}. The standardization of HIV-1 infection was achieved by means of the Alliance HIV-1 P24 antigen ELISA assay (PerkinElmer).

**Measurement of Extracellular ATP.** ATP release during cocultures or infection with HIV-1 were determined using the Enliten® ATP assay system (Promega) as described by the manufacturer. The luminescence was measured by integration over a 3-s time interval using the luminometer Fluostar OPTIMA (BMG Labtech).

**Hemifusion and cell-cell fusion assays.** HeLa Env⁺ and HeLa CD4⁺ cells were respectively stained for hemifusion analysis with 1 µM of 1,1'-dioctadecyl-3,3,3',3'-tetramethylindocarbocyanine perchlorate (DiIC₁₈(3), Invitrogen) and 10 µM of 5-chloromethylfluorescein diacetate (Cell Tracker Green CMFDA, Invitrogen) or for cell-cell fusion analysis, respectively with 10 µM of 5-chloromethylfluorescein diacetate (Cell Tracker Green CMFDA, Invitrogen) and 10 µM of 5-,6-,4-chloromethylbenzoyl aminotetramethyl rhodamine (CellTracker™ Orange CMTMR, Invitrogen). Cells were then cultured alone or together for 24-hours, at a 1:1 ratio in Dulbecco's modified Eagle's medium supplemented with 10% FCS, 2 mM L-glutamine and penicillin/streptomycin (Invitrogen) in the absence or presence of the indicated concentrations of purinergic receptor inhibitors. Effects of these inhibitors on hemifusion and cell-cell fusion mediated by the HIV-1 envelope were evaluated by fluorescence microscopy.

**β-galactosidase assay for virus-cell fusion.** HeLa cells stably transfected with CD4 as well as the LacZ gene under the control of the HIV-1 long terminal repeat (LTR) (HeLa-CD4) were selected in medium containing 500 mg/ml G418. Virus-cell fusion was evaluated using HeLa-CD4 cells. After 3 days of infection, cells were lysed, and β-galactosidase activity was measured using the enhanced ß-galactosidase assay kit (CPRG, Gene Therapy Systems).

**Plasma membrane depolarization detection.** PHA/IL-2 stimulated human lymphoblasts were stained with 300 nM DiBac4(3) (Invitrogen), for 20 min at 37 °C. Cells were then infected with HIV_{NDK} (at a multiplicity of infection of 1) for 1 hour. We then measured the increase of fluorescence intensity to reveal depolarized cells by flow cytometry. Propidium iodide uptake was used to exclude necrotic cells and evaluate plasma membrane depolarization in non-necrotic cells. Using the same procedure, we analyzed plasma membrane depolarization induced by the HIV-1 envelope.

**Patients.** Peripheral blood samples were obtained from healthy and HIV-1-infected individuals (all males, mean age 36 years), who were naive for highly active antiretroviral therapy (HAART) with a plasma viral load >20,000 copies/ml or who were receiving HAART with a viral load < 5,000 copies/ml. Plasma HIV-1 RNA levels were determined by the Abbott Real-time HIV-1 assay according to the manufacturer's instructions (Abbott Molecular). PBMCs of HIV-1 infected patients were obtained from residual samples intended to HIV RNA quantification for routine clinical management and were isolated by Ficoll/Hypaque (Amersham Biosciences AB, Uppsala, Sweden) centrifugation of heparinized blood from either healthy donors or HIV-seropositive individuals and fixed with 4% paraformaldehyde in PBS, pH 7.2. PBMCs from HAART treated patients that had undetectable virus in the plasma were used for ex-vivo experiments. The amounts of endogenous virus produced during these ex vivo experiments were monitored by using Vironostika HIV-1 Antigen assay according to manufacter's instructions (Biomerieux).

**Immunohistochemical analysis.** Biopsies from axillary lymph nodes were obtained in accordance with the Italian and EU legislations, after approval by the Institutional Review Board of the National Institute for Infectious Disease « Lazzaro Spalanzani ». Human tissue sections in paraffin were deparaffinized, rehydrated and subjected to high temperature antigen retrieval in 10 mM sodium citrate buffer pH 6.0. Endogenous peroxidase activity was blocked by 3% H₂O₂. Rabbit antibody against P2Y2 (Alomone labs) or Pyk2Y402* (Abcam) and biotinylated goat anti-rabbit IgG were incubated with tissue sections. Immunoreaction product obtained with a preformed horseradish peroxidase-conjugated streptavidin (Biogenex) was revealed using aminoethylcarbazole (AEC) as chromogenic substrates and 0.01% H₂O₂ (Biogenex). Sections were then counterstained in Mayer's acid hemalum. The lymph node and brain cortex sections from HIV-infected patients were semi-quantitatively evaluated by three independent observers using a light microscope. For each slide, a minimum of 10 fields was examined at 40X magnification. To describe the degree of the Pyk2 and P2Y2 expression on each specimen, an index comprised between 0 and 3 was assigned blind based on the percentage of positive cells present on the section (0 for 0 to 5%; 1 for 5 to 25%; 2 for 25 to 75%; and 3 for 75 to 100%).

**Statistical analysis.** To determine statistical significance, Student's *t*-test was used for calculation of *P* values.

### Results :

**ATP release from target cells participates in HIV-1 infection:** Because membrane stress can induce the release of ATP from mammalian cells, we speculated that HIV-1 infection might trigger ATP release from host cells. We found that infection of human cells with X4 tropic human immunodeficiency virus-1 (HIV_{NL43}), which relies on the expression of CD4 and CXCR4, the receptor and co-receptor of HIV-1, led to rapid release of ATP within the first two hours of infection (Fig. 1a). Inhibition of CXCR4 with its antagonist AMD3100 prevented ATP release, while addition of the non-toxic ATP-degrading enzyme, apyrase (Supplementary Information, Fig. S1a,b), prevented HIV-1 infection (Fig. 1b). Coculture of CD4/CXCR4-expressing cells with cells expressing the HIV-1 envelope glycoprotein complex (Env) at the plasma membrane also caused ATP release while the cells were engaged in conjugate formation (Fig. 1c). Apyrase abolished conjugate formation which was revealed by actin polymerization between two interacting cells (Fig. 1d). The release of ATP from cells constitutes a common response to shear stress and osmotic imbalance and can be mediated through mechanosensitive pannexin-1 hemichannels. Indeed, pannexin-1 was found to be enriched at the contact site between HIV-1-infected lymphoblasts (which express Env on the surface) and uninfected lymphoblasts (Fig. 1e) and to be associated with the virological synapse detected between the two interacting cells (Fig. 1f). Depletion of pannexin-1 by small interfering RNAs (siRNAs) inhibited the release of ATP from host cells during HIV-1 infection (Fig. 1g,h). In addition, pannexin-1 depletion or its chemical inhibition decreased HIV-1 infection and fusion between CD4/CXCR4 and Env-expressing cells (Fig. 1h, Supplementary Information S1c-e). Thus, extracellular ATP release plays a major role in Env-dependent interactions between HIV-1 and host cells.

**Purinergic receptors contribute to viral uptake:** Extracellular ATP constitutes a common danger signal that is sensed by distinct purinergic receptors. General inhibitors of purinergic receptors including suramin, pyridoxal-phosphate-6-azophenyl-2',4'-disulfonate (PPADS) and oxidized ATP (OxATP) blocked the replication of both X4-tropic HIV (e.g. HIV_{NDK}) and R5-tropic HIV (e.g. HIV_{BaL}) in activated T-lymphoblasts (Fig. 2a and Supplementary Information, Fig. S2a). Purinergic receptor antagonists also blocked the replication of R5 tropic HIV-1 in macrophages (Supplementary Information, Fig. S2b,c) or dendritic cells (Supplementary Information, Fig. S2d,e) and that of clinical HIV-1 isolates in C8166 T leukemia cells (Supplementary Information, Fig. S3a-c).

Cervical epithelial (HeLa) cells engineered to express CD4/CXCR4 as well as a Tat-inducible ß-galactosidase (ß-Gal) reporter expressed ß-Gal upon infection with a lymphotropic HIV-1 (HIV_{NL43WT}). Such cells also expressed ß-Gal upon infection by an isogenic virus in which the endogenous Env gene had been replaced by that of vesicular stomatitis virus (VSV) (HIV_{NL43□Env}) allowing the virus to infect cells that lack CD4/CXCR4. Purinergic receptor antagonists were more efficient in inhibiting ß-Gal induction by Env-expressing than that by VSV-pseudotyped HIV-1 (Fig. 2b and Supplementary Information, Fig. S2f), indicating that they mainly affect the receptor-dependent facet of HIV-1 infection. Purinergic receptor inhibition also reduced the hemifusion (which causes the exchange of lipids between interacting cells) and cell-to-cell fusion (syncytium formation) between CD4/CXCR4-positive cells and Env-expressing cells (Fig. 2c). Finally, purinergic receptor antagonists prevented the entry of HIV-1 into cells, as revealed by their inhibitory effects on the detection of HIV-genomes at the RNA (Fig. 2d) and DNA (Fig. 2e) levels within host cells. In contrast, purinergic receptor inhibition did not affect the replication of VSV-pseudotyped HIV-1 (Fig. 2d,e). These results demonstrate that purinergic receptors are critical for the Env-mediated internalization of HIV-1 into suitable target cells.

**Purinergic receptor P2Y2 modulates HIV-1 infection:** To identify the principal purinergic receptors involved in HIV-1 internalization, we systematically depleted the mRNAs coding for P2X1-, P2X4-, P2X7-, P2Y1-, P2Y2-, P2Y4- and P2Y6- receptors (which are expressed in HIV-1 target cells) by siRNA (Supplementary Information, Fig. S4a-g). Depletion of P2Y2 consistently resulted in the strongest reduction in Env-triggered syncytium formation (Supplementary Information, Fig. S4h). Likewise, immunoreactive P2Y2 was expressed at higher levels in lymphoid tissues (Fig. 3a,c), in the frontal cortex (Fig. 3b,d) as well as in circulating leukocytes from untreated HIV-1 carriers (Fig. 3e). P2Y2 was also found to be rapidly increased during HIV-1 infection in vitro (Fig. 3f). In addition, we detected by fluorescence microscopy the polarization of P2Y2 at the contact site between HIV-1-infected and uninfected lymphoblasts (Fig. 3g-k). The subcellular P2Y2 distribution revealed characteristic ring-like structures (Fig. 3i-k) that also contained the HIV-1 glycoprotein gp41 (Fig. 3k) and the coreceptor CD4, indicating that P2Y2 accumulated at the virological synapse that is formed between HIV-1-infected and uninfected lymphoblasts. Knockdown of P2Y2 (Fig. 3l) reduced HIV-1 infection, as well as Env-mediated hemifusion and fusion (Fig. 3m), suggesting that P2Y2 could control HIV-1 target cell infectivity. These results were confirmed by the increase in the fusogenic activity of HIV-1 envelope observed after transfection of target cells with P2Y2 cDNA (Fig. 3n and Supplementary Information, Fig. S4i). In addition, the fusion-inhibitory effect of a P2Y2-specific siRNA was overcome by transfection with a non-interferable P2Y2 cDNA but not with a non-functional P2Y2 mutant (P2Y2/4A) (Fig. 3n and Supplementary Information, Fig. S4i), underscoring the contribution of this purinergic receptor to HIV-1 infection.

**P2Y2 triggers plasma membrane depolarization to allow HIV-1 infection:** Infection with HIV-1 is associated with a transient depolarization of the plasma membrane (Fig. 4a). Transient abolition of the plasma membrane K⁺ gradient by culturing cells in a medium containing 70 mM KCl for 3 h strongly induced membrane depolarization (Fig. 4b,c) and enhanced the fusion of Env⁺ and CD4⁺/CXCR4⁺ cells that was reduced in the presence of the CXCR4 antagonist AMD3100 (Fig. 4d), demonstrating that plasma membrane depolarization of HIV-1 target cells is required for Env-dependent fusion events. Plasma membrane depolarization induced by HIV-1 infection was also abolished by addition of several distinct purinergic receptor antagonists, of the CXCR4 antagonist AMD3100 or ATP depletion with apyrase (Fig. 4e), indicating that purinergic receptors are involved in plasma membrane depolarization observed during HIV-1 infection. To determine the involvement of P2Y2 in this event, we transfected CD4⁺/CXCR4⁺ with a non-mutated P2Y2 cDNA or with a non-functional P2Y2 mutant (P2Y2/4A) and revealed that overexpression of P2Y2 - but not that of the non-functional P2Y2 mutant - increased the depolarization of the plasma membrane from CD4/CXCR4⁺ cells that were cocultured with Env⁺ cells (Fig. 4f). These results demonstrate that the Src homology domain 3 binding sites (PxxP) that is mutated in the carboxy-terminal tail of non-functional P2Y2 mutant (P2Y2/4A) is required for plasma membrane depolarization and contributes to Env-dependent fusion events.

**P2Y2 dependent activation of Pyk2 is required for HIV-1 infection:** P2Y2 (but not its mutant P2Y2/4A, see above) participates in the formation of a polyprotein complex that activates the proline-rich tyrosine kinase 2 (Pyk2). We found that the activating (auto)phosphorylation on tyrosine residue 402 of Pyk2 (Pyk2Y402*) increased during the formation of conjugates between Env⁺ and CD4/CXCR4⁺ HeLa cells (Fig. 5a). Moreover, Pyk2Y402* was enriched in ring-like structures at the contact site between such conjugates (Fig. 5b,d) and between HIV-1-infected lymphoblasts and uninfected lymphoblasts (Fig. 5c,e). The phosphorylation of Pyk2 was also detectable in lymph nodes from untreated HIV-1 carriers (Fig. 5f,h), in the frontal cortex from patients with HAE (Fig. 5g,i) and in circulating leukocytes from HIV-1 carriers, correlating with viral load (Fig. 5j). Furthermore highly active anti-retroviral therapy reduced immunodetectable Pyk2Y402* in leukocytes from HIV-1-infected donors (Fig. 5k). Depletion of P2Y2 reduced the phosphorylation of Pyk2 during conjugate formation (Fig. 5a) and inhibited the polarization of Pyk2Y402* at the contact site between cocultured Env⁺ and CD4/CXCR4⁺ cells (Fig. 5i). Pyk2 depletion also decreased HIV-1 infection and Env-mediated hemifusion and fusion (Fig. 5m,n). Taken together, these results indicate that Pyk2 is a critical effector of HIV-1 infection, operating downstream of P2Y2.

Depletion of each of the protein components of the hypothetical cascade delineated in this paper (pannexin-1→ATP→P2Y2→Pyk2) reduced infection by standard HIV-1 isolates (Fig. 1h, 2b-e, 3m, 5n) as well as by viruses that had been rendered resistant against reverse transcriptase and integrase inhibitors by selective point mutations (Fig. 6a). Similarly, purinergic receptor antagonists were able to inhibit infection by genetically engineered, therapy-resistant HIV-1 mutants (Fig. 6b) and tritherapy-selected viruses (Fig. 6c), consistent with the possibility that inhibition of the pannexin-1→ATP→ P2Y2→Pyk2 cascade can mediate antiretroviral effects under conditions in which conventional therapy fails. In addition, purinergic receptor antagonists reduced the death of activated CD4⁺ T lymphoblasts infected by HIV-1 (Fig. 6d), suggesting that interruption of P2Y2 signaling confers immunoprotective effects.

### Dicusssion:

While many early steps of infectious processes are associated with host membrane alterations, there are no studies that evaluate whether nucleotides are released as a consequence of viral infection and whether extracellular nucleotides might impact on the viral life cycle through signals affecting host cells. Here, we show that ATP is released into the extracellular milieu during the early steps of HIV-1 infection and that ATP is required for efficient viral uptake. During the initial stages of the infectious process, the release of ATP can determine viral clearance or the persistence of the infection by effects on the immune system. It was reported that massive ATP release during infection can deliver a "danger" signal to immune effectors. Extracellular ATP may activate the inflammosome, which participates in the caspase-1 dependent maturation and secretion of the pro-inflammatory cytokines IL-1beta, IL-18 and IL-33 or stimulate neutrophil migration and phagocytosis. Reportedly, extracellular ATP can inhibit infection by directly killing intracellular pathogens. Although ATP release can contribute to the clearance of pathogens from hosts, extracellular ATP can also participate in the survival of pathogens. Thus, a recent study revealed that OppA, the substrate-binding domain of *Mycoplasma hominis* permease increases the microbe's extracellular survival by inducing release of ATP from host cells. In addition, treatment of *Mycobacterium avium* infected cells with apyrase decreased intracellular survival of the bacilli. Our results differ from these studies in that they demonstrate that extracellular ATP released by HIV-1 host cells favors the establishment of HIV-1 infection. Thus, ATP that is rapidly released during the infectious process from HIV-1 target cells plays a role not only as a "danger" signal but also as a signaling molecule that contributes to viral uptake.

In this study, we report the identification of proteins involved in HIV-1-induced ATP release and in ATP-mediated signaling events that contribute to viral infection. Thus, we identified several novel proteins that are required for efficient HIV-1 infection of CD4⁺ host cells and showed that HIV-1 can stimulate ATP release into the extracellular milieu through pannexin-1 hemichannels, perhaps due to mechanical stress of the host cell membrane. ATP then acts in an autocrine/paracrine fashion to activate purinergic receptors, in particular P2Y2, which is a seven-transmembrane anchored G protein-coupled receptor and can induce the phosphorylation/ relocalization of the proline/tyrosine kinase Pyk2 to the virological synapse. Indeed, pannexin-1, P2Y2 and Pyk2 were all recruited to the contact site between Env-containing and CD4/CXCR4-containing membranes, suggesting that they act within the virological synapse to transduce signals that enhance membrane-to-membrane fusion, participate in the infectious process and facilitate viral transmission from cell to cell (Fig. 6e).

At present, the exact mechanisms by which P2Y2 modulates HIV-1 infection remain elusive. However, we found that after the interaction between HIV-1 envelope and its coreceptors, P2Y2 was activated and could directly interact with filamin A, a modulator of the actin cytoskeleton involved in the formation of stable receptor/coreceptor complexes. P2Y2 can also indirectly, through its SH3 binding (PxxP) site, control the phosphorylation of Pyk2 on tyrosine 402 (Pyk2Y402*), and this event might induce its phosphorylation/relocation to the virological synapse. Pyk2 regulates multiple signaling events that participate in the reorganization of the actin cytoskeleton and contribute to macrophage polarization and migration in response to chemokine stimulation. Pyk2 can control HIV-1 Env-mediated fusion. During HIV-1 infection, Pyk2 is phosphorylated after interactions between Env variants from X4- and R5 tropic strains and the Env receptors on HIV-1 target cells (CD4 and chemokine receptors). The activating (auto)phosphorylation of Pyk2 (Pyk2Y402*) that we detected during HIV-1 infection *in vitro* and *in vivo* might be induced in response to chemokine stimulation or by stimuli that elevate intracellular calcium concentrations and modulates the formation of a signaling complex that contains Src family kinases and actin interacting proteins. Here, we identified the ATP-mediated stimulation of the purinergic receptor P2Y2 as an upstream event required for the phosphorylation of Pyk2 (Pyk2Y402*). P2Y2 activation is known to modulate calcium influx after nucleotide binding and may also regulate Pyk2 activities by engaging in a polyprotein complex that contains the tyrosine kinase Src. Although we demonstrated that P2Y2 activation induced Pyk2Y402*, the precise mechanisms linking P2Y2 activation and polarization/phosphorylation of Pyk2 remain elusive. Moreover, future investigations must explore how plasma membrane depolarization, which is essential for HIV-1 infection, is induced upon the activation of P2Y2 and Pyk2.

Inhibition of any of the constituents of the molecular cascade that we delineated in this study (pannexin-1→ATP→P2Y2→Pyk2) could interrupt the HIV-1 life cycle at the level of viral entry. Thus, inhibition of pannexin hemichannels, enzymatic depletion of extracellular ATP, blockade of P2Y2 receptors or inhibition of Pyk2 kinase activity could be useful targets for the inhibition of HIV-1 infection, be it locally (for instance by application of topical gels) or systemically (ideally with orally available drugs).

## Claims

1. A blocking agent of a component of the purinergic signalling pathway selected from the group consisting of P2Y2 receptor antagonists and inhibitors of P2Y2 expression for use in the treatment or prevention of HIV-1 infection wherein the P2Y2 receptor antagonists are selected from the group consisting of antibodies and aptamers and the inhibitors of P2Y2 expression are selected from the group consisting of antisense RNA or DNA molecules, small inhibitory RNAs (siRNAs), short hairpin RNA and ribozymes.

2. A pharmaceutical composition comprising a blocking agent according to claim 1 for use in the treatment or prevention of HIV-1 infection.

3. The pharmaceutical composition for use according to claim 2 which is formulated as a topical formulation.

4. The pharmaceutical composition for use according to claim 3 which is a gel.

## Patentansprüche

1. Blockierungsmittel einer Komponente des purinergen Signalwegs, ausgewählt aus der Gruppe bestehend aus P2Y2-Rezeptorantagonisten und Inhibitoren der P2Y2-Expression, zur Verwendung in der Behandlung oder Vorbeugung von HIV-1-Infektion, wobei die P2Y2-Rezeptorantagonisten ausgewählt sind aus der Gruppe bestehend aus Antikörpern und Aptameren und die Inhibitoren der P2Y2-Expression ausgewählt sind aus der Gruppe bestehend aus Antisense-RNA- oder -DNA-Molekülen, kleinen inhibitorischen RNAs (small inhibitory RNAs, siRNAs), short hairpin RNA und Ribozymen.

2. Pharmazeutische Zusammensetzung, umfassend ein Blockierungsmittel gemäß Anspruch 1 zur Verwendung in der Behandlung oder der Vorbeugung von HIV-1-Infektion.

3. Pharmazeutische Zusammensetzung zur Verwendung gemäß Anspruch 2, welche als topische Formulierung formuliert ist.

4. Pharmazeutische Zusammensetzung zur Verwendung gemäß Anspruch 3, welche ein Gel ist.

## Revendications

1. Agent de blocage d'un composant de la voie de signalisation purinergique choisi dans le groupe constitué d'antagonistes du récepteur P2Y2 et d'inhibiteurs de l'expression du P2Y2 pour une utilisation dans le traitement ou la prévention d'une infection par le VIH-1, les antagonistes du récepteur P2Y2 étant choisis dans le groupe constitué d'anticorps et d'aptamères et les inhibiteurs de l'expression du P2Y2 étant choisis dans le groupe constitué de molécules d'ARN ou d'ADN antisens, de petits ARN inhibiteurs (ARNsi), de petits ARN en épingle à cheveux et de ribozymes.

2. Composition pharmaceutique comprenant un agent de blocage selon la revendication 1 pour une utilisation dans le traitement ou la prévention d'une infection par le VIH-1.

3. Composition pharmaceutique pour une utilisation selon la revendication 2, qui est formulée sous la forme d'une formulation topique.

4. Composition pharmaceutique pour une utilisation selon la revendication 3 qui est un gel.


### SEQUENCE LISTING

<110> INSERM
<120> BLOCKING AGENTS OF THE PURINERGIC SIGNALLING PATHWAY FOR USE IN THE TREATMENT OR PREVENTION OF HIV-1 INFECTION
<130> BIO09429EP
<160> 36
<170> PatentIn version 3.3
<210> 1
   <211> 10
   <212> PRT
   <213> Artificial
<220>
   <223> pannexin 1 mimetic blocking peptide
<400> 1
   Trp Arg Gln Ala Ala Phe Val Asp Ser Tyr
   1               5                   10
<210> 2
   <211> 19
   <212> RNA
   <213> Artificial
<220>
   <223> siRNA
<400> 2
   gcuccaaggu augaacaua 19
<210> 3
   <211> 19
   <212> RNA
   <213> Artificial
<220>
   <223> siRNA
<400> 3
   gcuccaaggu augaacaua 19
<210> 4
   <211> 19
   <212> RNA
   <213> Artificial
<220>
   <223> siRNA
<400> 4
   guggcaguca guaaccaua 19
<210> 5
   <211> 19
   <212> RNA
   <213> Artificial
<220>
   <223> siRNA
<400> 5
   ggaaaacucc cucuucguc 19
<210> 6
   <211> 19
   <212> RNA
   <213> Artificial
<220>
   <223> siRNA
<400> 6
   gcugucgcuc ccauauuua 19
<210> 7
   <211> 19
   <212> RNA
   <213> Artificial
<220>
   <223> siRNA
<400> 7
   ggucucauua uauauuggg 19
<210> 8
   <211> 25
   <212> RNA
   <213> Artificial
<220>
   <223> siRNA
<400> 8
   auuaucacca gguaaaugga uuucc 25
<210> 9
   <211> 19
   <212> RNA
   <213> Artificial
<220>
   <223> siRNA
<400> 9
   ggauagaaga uguguuggg 19
<210> 10
   <211> 19
   <212> RNA
   <213> Artificial
<220>
   <223> siRNA
<400> 10
   ggcuguaacu uauacuaaa 19
<210> 11
   <211> 19
   <212> RNA
   <213> Artificial
<220>
   <223> siRNA
<400> 11
   ggacaguagc ugcucuacu 19
<210> 12
   <211> 19
   <212> RNA
   <213> Artificial
<220>
   <223> siRNA
<400> 12
   gaaaagagga agaagcacc 19
<210> 13
   <211> 19
   <212> RNA
   <213> Artificial
<220>
   <223> siRNA
<400> 13
   gaacuucaag caacugcug 19
<210> 14
   <211> 18
   <212> RNA
   <213> Artificial
<220>
   <223> siRNA
<400> 14
   ggugcucaca aaaauaca 18
<210> 15
   <211> 19
   <212> RNA
   <213> Artificial
<220>
   <223> siRNA
<400> 15
   cuguaccggu cauacguaa 19
<210> 16
   <211> 19
   <212> RNA
   <213> Artificial
<220>
   <223> siRNA
<400> 16
   gcugaucggc aucauugaa 19
<210> 17
   <211> 19
   <212> RNA
   <213> Artificial
<220>
   <223> siRNA
<400> 17
   ggcgguucuu caaggauau 19
<210> 18
   <211> 19
   <212> RNA
   <213> Artificial
<220>
   <223> siRNA
<400> 18
   gccgguaugc cgguuaagu 19
<210> 19
   <211> 22
   <212> DNA
   <213> Artificial
<220>
   <223> primer
<400> 19
   gctggtgcgt aataagaagg tg 22
<210> 20
   <211> 20
   <212> DNA
   <213> Artificial
<220>
   <223> primer
<400> 20
   atgaggccgc tcgaggtctg 20
<210> 21
   <211> 22
   <212> DNA
   <213> Artificial
<220>
   <223> primer
<400> 21
   gataccagct caggaggaaa ac 22
<210> 22
   <211> 23
   <212> DNA
   <213> Artificial
<220>
   <223> primer
<400> 22
   gcatcataaa tgcacgactt gag 23
<210> 23
   <211> 23
   <212> DNA
   <213> Artificial
<220>
   <223> primer
<400> 23
   tgataaaagt cttcgggatc cgt 23
<210> 24
   <211> 23
   <212> DNA
   <213> Artificial
<220>
   <223> primer
<400> 24
   tggacaaatc tgtgaagtcc atc 23
<210> 25
   <211> 21
   <212> DNA
   <213> Artificial
<220>
   <223> primer
<400> 25
   cttggtgctg attctgggct g 21
<210> 26
   <211> 21
   <212> DNA
   <213> Artificial
<220>
   <223> primer
<400> 26
   gctcgggaga gtctccttct g 21
<210> 27
   <211> 21
   <212> DNA
   <213> Artificial
<220>
   <223> primer
<400> 27
   ccgctcgctg gacctcagct g 21
<210> 28
   <211> 21
   <212> DNA
   <213> Artificial
<220>
   <223> primer
<400> 28
   ctcactgctg cccaacacat c 21
<210> 29
   <211> 20
   <212> DNA
   <213> Artificial
<220>
   <223> primer
<400> 29
   cgctgcccac cctcatctac 20
<210> 30
   <211> 21
   <212> DNA
   <213> Artificial
<220>
   <223> primer
<400> 30
   ccgagtggtg tcatggcaca g 21
<210> 31
   <211> 21
   <212> DNA
   <213> Artificial
<220>
   <223> primer
<400> 31
   aaccttgctc tggctgacct g 21
<210> 32
   <211> 20
   <212> DNA
   <213> Artificial
<220>
   <223> primer
<400> 32
   gcaggcactg ggttgtcacg 20
<210> 33
   <211> 22
   <212> DNA
   <213> Artificial
<220>
   <223> primer
<400> 33
   accggtcata cgtaagaacg ag 22
<210> 34
   <211> 21
   <212> DNA
   <213> Artificial
<220>
   <223> primer
<400> 34
   gcagaattgg ggcacttcag c 21
<210> 35
   <211> 21
   <212> DNA
   <213> Artificial
<220>
   <223> primer
<400> 35
   caatgcatcc tgcaccacca a 21
<210> 36
   <211> 21
   <212> DNA
   <213> Artificial
<220>
   <223> primer
<400> 36
   gtcattgaga gcaatgccag c 21